# EUROPEAN PATENT SPECIFICATION

(11) **EP 3 464 565 B1**
(45) Date of publication and mention of the grant of the patent: **28.10.2026**
(21) Application number: 17803614.1
(22) Date of filing: 25.05.2017
(51) Int. Cl.: C12N 5/0775, C12N 5/02, A61K 35/12, A61K 35/15, C12M 1/12, C12N 5/0789

(54) **CELL EXPANSION**
ZELLEXPANSION
EXPANSION CELLULAIRE

(30) Priority: 25.05.2016 US 201662341523 P
(43) Date of publication of application: 10.04.2019
(73) Proprietor: Terumo BCT, Inc., Lakewood, CO 80215 (US)
(72) Inventor: JONES, Mark E., Littleton Colorado 80127 (US)
(74) Representative: J A Kemp LLP
(86) International application number: PCT/US2017/034544
(87) International publication number: WO 2017/205667

(56) References cited:
- WO-A1-00/46349
- US-A1- 2006 073 591
- US-A1- 2012 308 531
- US-A1- 2013 330 306
- US-A1- 2016 362 659
- US-B2- 8 080 417
- YUE ZHANG ET AL: "Co-culture of Umbilical Cord Blood CD34+ Cells with Human Mesenchymal Stem Cells", TISSUE ENGINEERING, vol. 12, no. 8, 1 August 2006 (2006-08-01), pages 2161 - 2170, XP055064299, ISSN: 1076-3279, DOI: 10.1089/ten.2006.12.2161
- MICHAEL R. DORAN ET AL: "Bioreactor for Blood Product Production", CELL TRANSPLANTATION, vol. 21, no. 6, 1 June 2012 (2012-06-01), US, pages 1235 - 1244, XP055644897, ISSN: 0963-6897, DOI: 10.3727/096368911X627363
- CAO XUE ET AL: "The hollow fiber bioreactor as a stroma-supported, serum-free ex vivo expansion platform for human umbilical cord blood cells", BIOTECHNOLOGY JOURNAL, vol. 9, no. 7, 24 February 2014 (2014-02-24), DE, pages 980 - 989, XP055524404, ISSN: 1860-6768, DOI: 10.1002/biot.201300320
- BARI SUDIPTO ET AL: "Expansion and Homing of Umbilical Cord Blood Hematopoietic Stem and Progenitor Cells for Clinical Transplantation", BIOLOGY OF BLOOD AND MARROW TRANSPLANTATION, KLUGE CARDEN JENNINGS PUBLISHING, CHARLOTTESVILLE, VA, US, vol. 21, no. 6, 30 December 2014 (2014-12-30), pages 1008 - 1019, XP029155489, ISSN: 1083-8791, DOI: 10.1016/J.BBMT.2014.12.022
- NOLD PHILIPP ET AL: "Good manufacturing practice-compliant animal-free expansion of human bone marrow derived mesenchymal stroma cells in a closed hollow-fiber-based bioreactor", BIOCHEMICAL AND BIOPHYSICAL RESEARCH COMMUNICATIONS, ELSEVIER, AMSTERDAM, NL, vol. 430, no. 1, 9 November 2012 (2012-11-09), pages 325 - 330, XP028962856, ISSN: 0006-291X, DOI: 10.1016/J.BBRC.2012.11.001

## Description

### Background

Cell Expansion Systems (CES's) may be used to expand and differentiate a variety of cell types that may be used for both research and therapeutic purposes. As one example, CD34+ progenitor cells hematological progenitor stem cells (HSC's) have been identified as possible treatments in diseases such as cancers (e.g., lymphoma, leukemia, myeloma, etc.). HSC's may be collected from bone marrow, cord blood, and or peripheral blood. It has been suggested that a minimum number of HSC's should be given as an effective dose. Accordingly, the HSC's may be grown from an initial amount to at least an amount that may be considered an effective dose.

Embodiments have been made in light of these and other considerations. However, the relatively specific problems discussed above do not limit the applicability of the embodiments of the present disclosure.
CAO XUE ET AL: "The hollow fiber bioreactor as a stroma-supported, serum-free ex vivo expansion platform for human umbilical cord blood cells", BIOTECHNOLOGY JOURNAL, vol. 9, no. 7, 24 February 2014 (2014-02-24), pages 980-989, DE ISSN: 1860-6768, DOl: 10.1002/biot.201300320, discloses a hollow fiber bioreactor as a stroma-supported, serum-free ex vivo expansion platform for human umbilical cord blood cells.

### Summary

The invention is defined in claim 1. Further aspects and preferred embodiments are defined in the dependent claims. Any aspects, embodiments and examples of the present disclosure which do not fall under the scope of the appended claims do not form part of the invention and are merely provided for illustrative purposes.

The summary is provided to introduce aspects of some embodiments in a simplified form, and is not intended to identify key or essential elements, nor is it intended to limit the scope of the claims.

Embodiments relate to cell expansion systems (CES's) and methods of growing cells in a bioreactor of a cell expansion system. Embodiments provide methods for expanding cells in accordance with appended claim 1.

### Brief Description of the Drawings

Non-limiting and non-exhaustive embodiments are described with reference to the following figures.
FIG. 1 depicts a perspective view of a hollow fiber bioreactor, usable with embodiments.
FIG. 2 illustrates a perspective view of a cell expansion system with a premounted fluid conveyance device, usable with embodiments.
FIG. 3 depicts a perspective view of a housing of a cell expansion system, usable with embodiments.
FIG. 4 illustrates a perspective view of a premounted fluid conveyance device, usable with embodiments.
FIG. 5 depicts a schematic of a cell expansion system, usable with embodiments.
FIG. 6 illustrates a schematic of another cell expansion system, usable with embodiments.
FIG. 7 illustrates a flow of a process for expanding cells that is useful for understanding the invention.
FIG. 8 illustrates a flow of a process for expanding cells according to an embodiment.
FIG. 9 illustrates a flow of a process for expanding cells according to another embodiment.
FIGS. 10A-D illustrate a cross section of a hollow fiber during rotation of a bioreactor, usable with embodiments.
FIG. 11 illustrates components of a computing system that may be used to implement embodiments.
FIG. 12 illustrates a bar graph of the number of cells grown according to one embodiment for expansion of CD34+ cells in a bioreactor system.
FIG. 13 illustrates a graph showing metabolic profile according to one embodiment for expansion of CD34+ cells.
FIG. 14 illustrates a graph showing metabolic rates according to one embodiment for expansion of CD34+ cells.
FIG. 15 illustrates a graph showing cell counts according to one embodiment for expansion of CD34+ cells.
FIG. 16 illustrates a graph showing cell counts according to another embodiment for expansion of CD34+ cells.
FIG. 17 illustrates a graph showing cell counts according to another embodiment for expansion of CD34+ cells.
FIG. 18 illustrates a bar graph showing different types of biomarkers on cells grown according to embodiments.

### Detailed Description

The principles of the present disclosure may be further understood by reference to the following detailed description and the embodiments depicted in the accompanying drawings. It should be understood that although specific features are shown and described below with respect to detailed embodiments, the present disclosure is not limited to the embodiments described below.

Reference will now be made in detail to the embodiments illustrated in the accompanying drawings and described below. Wherever possible, the same reference numerals are used in the drawings and the description to refer to the same or like parts.

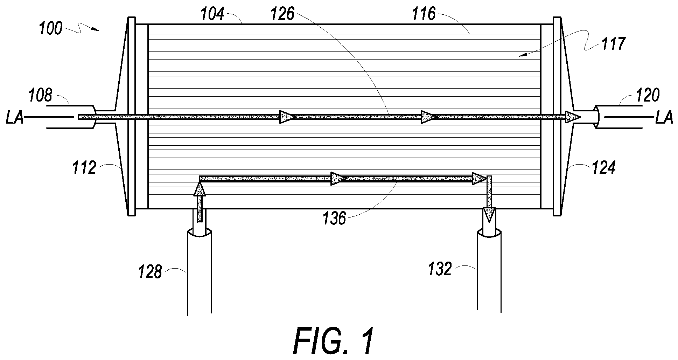

Referring to FIG. 1, an example of a hollow fiber bioreactor 100, which may be used with the present disclosure is shown in front side elevation view. Hollow fiber bioreactor 100 has a longitudinal axis LA-LA and includes chamber housing 104. In at least one embodiment, chamber housing 104 includes four openings or ports: intracapillary (IC) inlet port 108, IC outlet port 120, extracapillary (EC) inlet port 128, and EC outlet port 132.

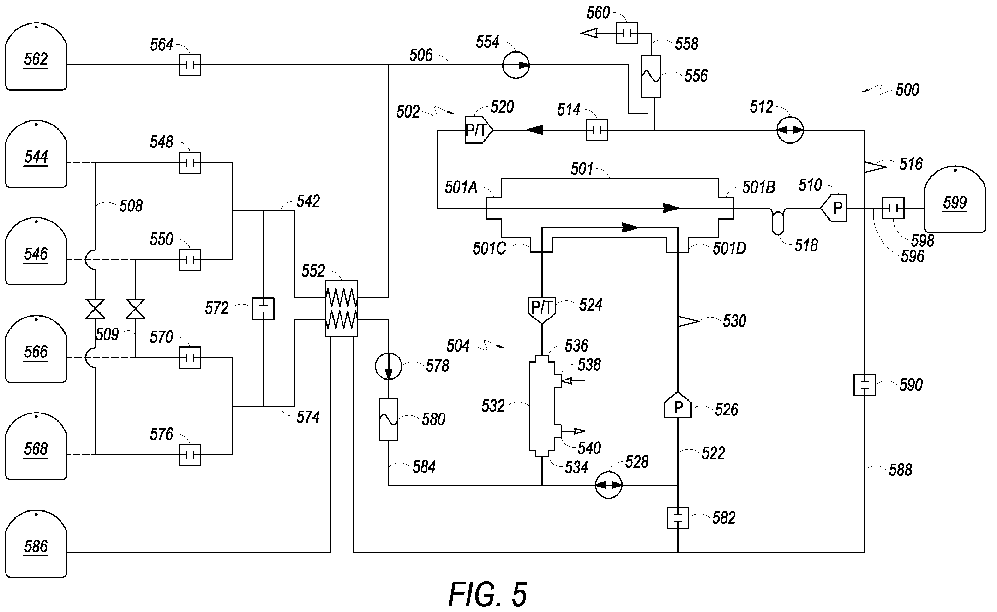

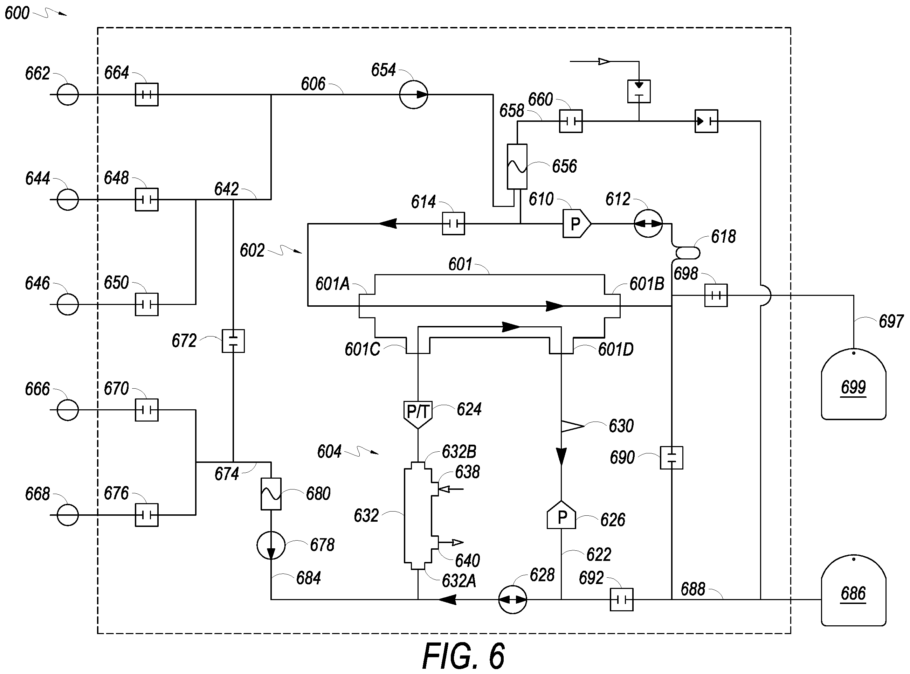

According to embodiments of the present disclosure, fluid in a first circulation path enters hollow fiber bioreactor 100 through IC inlet port 108 at a first longitudinal end 112 of the hollow fiber bioreactor 100, passes into and through the intracapillary side (referred to in various embodiments as the intracapillary ("IC") side or "IC space" of a hollow fiber membrane) of a plurality of hollow fibers 116, and out of hollow fiber bioreactor 100 through IC outlet port 120 located at a second longitudinal end 124 of the hollow fiber bioreactor 100. The fluid path between the IC inlet port 108 and the IC outlet port 120 defines the IC portion 126 of the hollow fiber bioreactor 100. Fluid in a second circulation path flows in the hollow fiber bioreactor 100 through EC inlet port 128, comes in contact with the extracapillary side or outside (referred to as the "EC side" or "EC space" of the membrane) of the hollow fibers 116, and exits hollow fiber bioreactor 100 via EC outlet port 132. The fluid path between the EC inlet port 128 and the EC outlet port 132 comprises the EC portion 136 of the hollow fiber bioreactor 100. Fluid entering hollow fiber bioreactor 100 via the EC inlet port 128 may be in contact with the outside of the hollow fibers 116. Small molecules (e.g., ions, water, oxygen, lactate, etc.) may diffuse through the hollow fibers 116 from the interior or IC space of the hollow fiber to the exterior or EC space, or from the EC space to the IC space. Large molecular weight molecules, such as growth factors, may be too large to pass through the hollow fiber membrane, and remain in the IC space of the hollow fibers 116. The media may be replaced as needed, in embodiments. Media may also be circulated through an oxygenator or gas transfer module to exchange gasses as needed (see e.g., cell expansion systems 500 (FIG. 5) and 600 (FIG. 6)). Cells may be contained within a first circulation path and/or a second circulation path, as described below, and may be on either the IC side and/or EC side of the membrane, according to embodiments.

The material used to make the hollow fiber membrane may be any biocompatible polymeric material which is capable of being made into hollow fibers. One material which may be used is a synthetic polysulfone-based material, according to an embodiment of the present disclosure. In order for the cells to adhere to the surface of the hollow fibers, the surface is modified, by coating at least the cell growth surface with fibronectin and optionally collagen, and optionally by exposing the surface to radiation. Gamma treating the membrane surface may allow for attachment of adherent cells, without additionally coating the membrane with fibronectin or the like (in examples not according to the invention). Bioreactors made of gamma treated membranes may be reused. Additional coatings and/or treatments for cell attachment may be used in accordance with embodiments of the present disclosure.

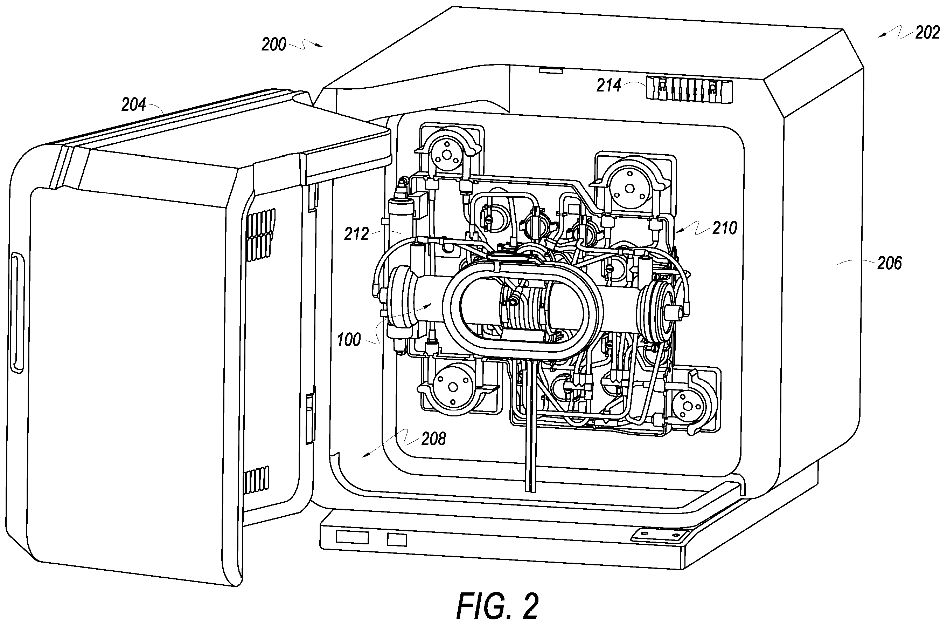

Turning to FIG. 2, an embodiment of a cell expansion system 200 with a premounted fluid conveyance assembly is shown in accordance with embodiments of the present disclosure. The CES 200 includes a cell expansion machine 202 that comprises a hatch or closable door 204 for engagement with a back portion 206 of the cell expansion machine 202. An interior space 208 within the cell expansion machine 202 includes features adapted for receiving and engaging a premounted fluid conveyance assembly 210 that includes a bioreactor 100. The premounted fluid conveyance assembly 210 may be detachably-attachable to the cell expansion machine 202 to facilitate relatively quick exchange of a new or unused premounted fluid conveyance assembly 210 at a cell expansion machine 202 for a used premounted fluid conveyance assembly 210 at the same cell expansion machine 202. A single cell expansion machine 202 may be operated to grow or expand a first set of cells using a first premounted fluid conveyance assembly 210 and, thereafter, may be used to grow or expand a second set of cells using a second premounted fluid conveyance assembly 210 without needing to be sanitized between interchanging the first premounted fluid conveyance assembly 210 for the second premounted fluid conveyance assembly 210. The premounted fluid conveyance assembly includes a bioreactor 100 and an oxygenator or gas transfer module 212. Tubing guide slots are shown as 214 for receiving various media tubing connected to premounted fluid conveyance assembly 210, according to embodiments.

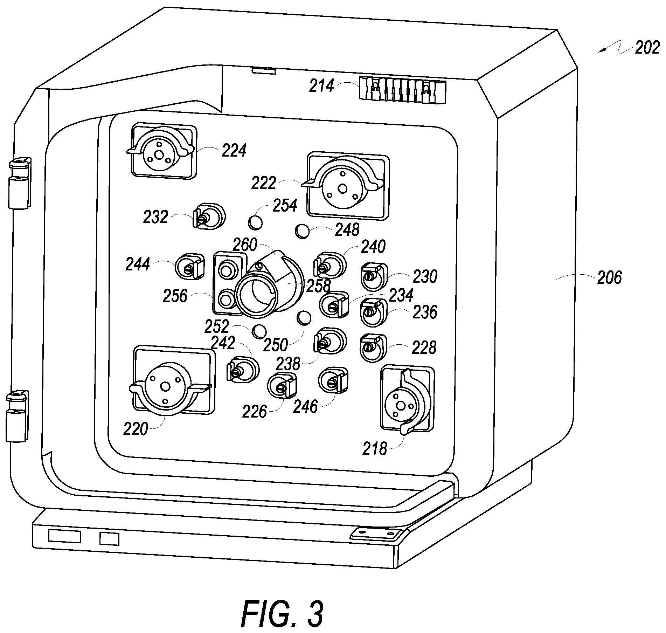

Next, FIG. 3 illustrates the back portion 206 of cell expansion machine 202 prior to detachably-attaching a premounted fluid conveyance assembly 210 (FIG. 2), in accordance with embodiments of the present disclosure. The closable door 204 (shown in FIG. 2) is omitted from FIG. 3. The back portion 206 of the cell expansion machine 202 includes a number of different structures for working in combination with elements of a premounted fluid conveyance assembly 210. More particularly, the back portion 206 of the cell expansion machine 202 includes a plurality of peristaltic pumps for cooperating with pump loops on the premounted fluid conveyance assembly 210, including the IC circulation pump 218, the EC circulation pump 220, the IC inlet pump 222, and the EC inlet pump 224. In addition, the back portion 206 of the cell expansion machine 202 includes a plurality of valves, including the IC circulation valve 226, the reagent valve 228, the IC media valve 230, the air removal valve 232, the cell inlet valve 234, the wash valve 236, the distribution valve 238, the EC media valve 240, the IC waste valve 242, the EC waste valve 244, and the harvest valve 246. Several sensors are also associated with the back portion 206 of the cell expansion machine 202, including the IC outlet pressure sensor 248, the combination IC inlet pressure and temperature sensors 250, the combination EC inlet pressure and temperature sensors 252, and the EC outlet pressure sensor 254. Also shown is an optical sensor 256 for an air removal chamber.

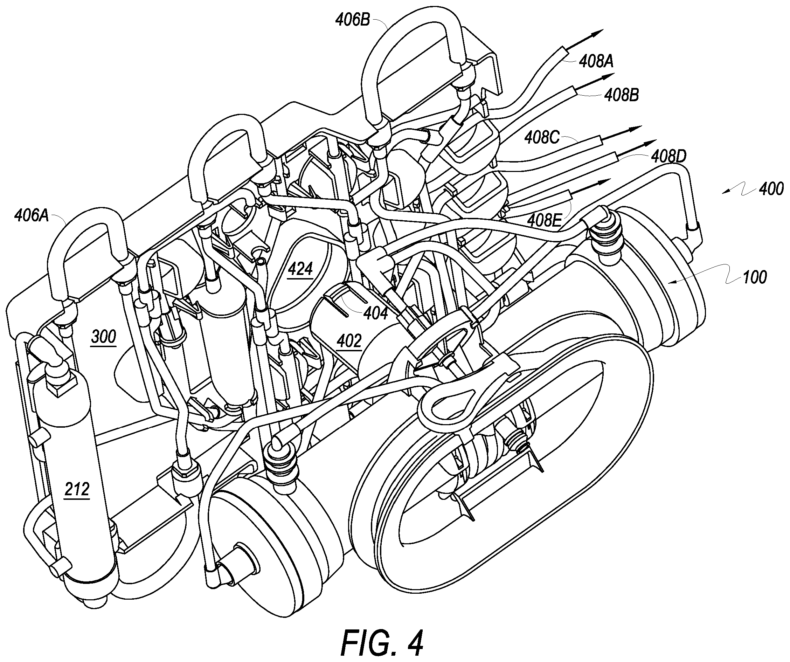

In accordance with embodiments, a shaft or rocker control 258 for rotating the bioreactor 100 is shown in FIG. 3. Shaft fitting 260 associated with the shaft or rocker control 258 allows for proper alignment of a shaft access aperture, see e.g., 424 (FIG. 4) of a tubing-organizer, see e.g., 300 (FIG. 4) of a premounted conveyance assembly 210 or 400 with the back portion 206 of the cell expansion machine 202. Rotation of shaft or rocker control 258 imparts rotational movement to shaft fitting 260 and bioreactor 100. Thus, when an operator or user of the CES 200 attaches a new or unused premounted fluid conveyance assembly 400 (FIG. 4) to the cell expansion machine 202, the alignment is a relatively simple matter of properly orienting the shaft access aperture 424 (FIG. 4) of the premounted fluid conveyance assembly 210 or 400 with the shaft fitting 260.

Turning to FIG. 4, a perspective view of a detachably-attachable premounted fluid conveyance assembly 400 is shown. The premounted fluid conveyance assembly 400 may be detachably-attachable to the cell expansion machine 202 to facilitate relatively quick exchange of a new or unused premounted fluid conveyance assembly 400 at a cell expansion machine 202 for a used premounted fluid conveyance assembly 400 at the same cell expansion machine 202. As shown in FIG. 4, the bioreactor 100 may be attached to a bioreactor coupling that includes a shaft fitting 402. The shaft fitting 402 includes one or more shaft fastening mechanisms, such as a biased arm or spring member 404 for engaging a shaft, e.g., 258 (shown in FIG. 3), of the cell expansion machine 202.

In embodiments, the shaft fitting 402 and the spring member 404 connect to mechanisms of a cell expansion system that rotate the bioreactor 100. For example, in some embodiments, the cell expansion system may be part of a QUANTUM^{®} Cell Expansion System (CES), manufactured by Terumo BCT, Inc. of Lakewood, CO, which provides for rotation of a bioreactor. Examples of cell expansion systems that provide for rotation of the bioreactor are described in at least: U.S. Patent No. 8,399,245, issued March 19, 2013, entitled "ROTATION SYSTEM FOR CELL GROWTH CHAMBER OF A CELL EXPANSION SYSTEM AND METHOD OF USE THEREFOR;" U.S. Patent No. 8,809,043, issued February 13, 2013, entitled "ROTATION SYSTEM FOR CELL GROWTH CHAMBER OF A CELL EXPANSION SYSTEM AND METHOD OF USE THEREFOR;" and U.S. Patent No. 9,057,045, issued June 16, 2015, entitled "METHOD OF LOADING AND DISTRIBUTING CELLS IN A BIOREACTOR OF A CELL EXPANSION SYSTEM".

According to embodiments, the premounted fluid conveyance assembly 400 includes tubing 408A, 408B, 408C, 408D, 408E, etc., and various tubing fittings to provide the fluid paths shown in FIGS. 5 and 6, as discussed below. Pump loops 406A, 406B, and 406C are also provided for the pump(s). In embodiments, although the various media may be provided at the site where the cell expansion machine 202 is located, the premounted fluid conveyance assembly 400 may include sufficient tubing length to extend to the exterior of the cell expansion machine 202 and to enable welded connections to tubing associated with the media bags, according to embodiments.

FIG. 5 illustrates a schematic of an embodiment of a cell expansion system 500, and FIG. 6 illustrates a schematic of another embodiment of a cell expansion system 600. In the embodiments shown in FIGS. 5 and 6, and as described below, the cells are grown in the IC space. However, the disclosure is not limited to such examples and may in other embodiments provide for cells to be grown in the EC space. In yet other embodiments, such as when co-culturing cells, first cells may be grown in the EC space, while second cells may be grown in the IC space. Co-culturing of cells may also be performed by growing first cells and second cells in the EC space, or growing first cells and second cells in the IC space.

FIG. 5 illustrates a CES 500, which includes first fluid circulation path 502 (also referred to as the "intracapillary loop" or "IC loop") and second fluid circulation path 504 (also referred to as the "extracapillary loop" or "EC loop"), according to embodiments. First fluid flow path 506 may be fluidly associated with hollow fiber bioreactor 501 to form, at least in part, first fluid circulation path 502. Fluid flows into hollow fiber bioreactor 501 through IC inlet port 501A, through hollow fibers in hollow fiber bioreactor 501, and exits via IC outlet port 501B. Pressure gauge 510 measures the pressure of media leaving hollow fiber bioreactor 501. Media flows through IC circulation pump 512 which may be used to control the rate of media flow/rate of fluid circulation. IC circulation pump 512 may pump the fluid in a first direction (e.g., clockwise) or second direction opposite the first direction (e.g., counter clockwise). Exit port 501B may be used as an inlet in the reverse direction. Media entering the IC loop 502 may then enter through valve 514. As those skilled in the art will appreciate, additional valves and/or other devices may be placed at various locations to isolate and/or measure characteristics of the media along portions of the fluid paths. Accordingly, it is to be understood that the schematic shown represents one possible configuration for various elements of the CES 500, and modifications to the schematic shown are within the scope of the one or more present embodiments.

With regard to the IC loop 502, samples of media may be obtained from sample port 516 or sample coil 518 during operation. Pressure/temperature gauge 520 disposed in first fluid circulation path 502 allows detection of media pressure and temperature during operation. Media then returns to IC inlet port 501A to complete fluid circulation path 502. Cells grown/expanded in hollow fiber bioreactor 501 may be flushed out of hollow fiber bioreactor 501 into harvest bag 599 through valve 598 or redistributed within the hollow fibers for further growth.

Fluid in second fluid circulation path 504 enters hollow fiber bioreactor 501 via EC inlet port 501C, and leaves hollow fiber bioreactor 501 via EC outlet port 501D. Media in the EC loop 504 may be in contact with the outside of the hollow fibers in the hollow fiber bioreactor 501, thereby allowing diffusion of small molecules into and out of the hollow fibers.

Pressure/temperature gauge 524 disposed in the second fluid circulation path 504 allows the pressure and temperature of media to be measured before the media enters the EC space of hollow fiber bioreactor 501. Pressure gauge 526 allows the pressure of media in the second fluid circulation path 504 to be measured after it leaves hollow fiber bioreactor 501. With regard to the EC loop, samples of media may be obtained from sample port 530 or a sample coil during operation.

In embodiments, after leaving EC outlet port 501D of hollow fiber bioreactor 501, fluid in second fluid circulation path 504 passes through EC circulation pump 528 to oxygenator or gas transfer module 532. EC circulation pump 528 may also pump the fluid in opposing directions. Second fluid flow path 522 may be fluidly associated with oxygenator or gas transfer module 532 via oxygenator inlet port 534 and oxygenator outlet port 536. In operation, fluid media flows into oxygenator or gas transfer module 532 via oxygenator inlet port 534, and exits oxygenator or gas transfer module 532 via oxygenator outlet port 536. Oxygenator or gas transfer module 532 adds oxygen to and removes bubbles from media in the CES 500. In various embodiments, media in second fluid circulation path 504 may be in equilibrium with gas entering oxygenator or gas transfer module 532. The oxygenator or gas transfer module 532 may be any appropriately sized oxygenator or gas transfer device. Air or gas flows into oxygenator or gas transfer module 532 via filter 538 and out of oxygenator or gas transfer device 532 through filter 540. Filters 538 and 540 reduce or prevent contamination of oxygenator or gas transfer module 532 and associated media. Air or gas purged from the CES 500 during portions of a priming sequence may vent to the atmosphere via the oxygenator or gas transfer module 532.

In the configuration depicted for CES 500, fluid media in first fluid circulation path 502 and second fluid circulation path 504 flows through hollow fiber bioreactor 501 in the same direction (a co-current configuration). The CES 500 may also be configured to flow in a counter-current configuration.

In accordance with at least one embodiment, media, including cells (from bag 562), and fluid media from bag 546 may be introduced to first fluid circulation path 502 via first fluid flow path 506. Fluid container 562 (e.g., Cell Inlet Bag or Saline Priming Fluid for priming air out of the system) may be fluidly associated with the first fluid flow path 506 and the first fluid circulation path 502 via valve 564.

Fluid containers, or media bags, 544 (e.g., Reagent) and 546 (e.g., IC Media) may be fluidly associated with either first fluid inlet path 542 via valves 548 and 550, respectively, or second fluid inlet path 574 via valves 548, 550, and 570. First and second sterile sealable input priming paths 508 and 509 are also provided. An air removal chamber (ARC) 556 may be fluidly associated with first circulation path 502. The air removal chamber 556 may include one or more ultrasonic sensors including an upper sensor and lower sensor to detect air, a lack of fluid, and/or a gas/fluid interface, e.g., an air/fluid interface, at certain measuring positions within the air removal chamber 556. For example, ultrasonic sensors may be used near the bottom and/or near the top of the air removal chamber 556 to detect air, fluid, and/or an air/fluid interface at these locations. Embodiments provide for the use of numerous other types of sensors without departing from the scope of the present disclosure. For example, optical sensors may be used in accordance with embodiments of the present disclosure. Air or gas purged from the CES 500 during portions of the priming sequence or other protocols may vent to the atmosphere out air valve 560 via line 558 that may be fluidly associated with air removal chamber 556.

EC media (from bag 568) or wash solution (from bag 566) may be added to either the first or second fluid flow paths. Fluid container 566 may be fluidly associated with valve 570 that may be fluidly associated with first fluid circulation path 502 via distribution valve 572 and first fluid inlet path 542. Alternatively, fluid container 566 may be fluidly associated with second fluid circulation path 504 via second fluid inlet path 574 and EC inlet path 584 by opening valve 570 and closing distribution valve 572. Likewise, fluid container 568 may be fluidly associated with valve 576 that may be fluidly associated with first fluid circulation path 502 via first fluid inlet path 542 and distribution valve 572. Alternatively, fluid container 568 may be fluidly associated with second fluid inlet path 574 by opening valve 576 and closing valve distribution 572. An optional heat exchanger 552 may be provided for media reagent or wash solution introduction.

In the IC loop, fluid may be initially advanced by the IC inlet pump 554. In the EC loop, fluid may be initially advanced by the EC inlet pump 578. An air detector 580, such as an ultrasonic sensor, may also be associated with the EC inlet path 584.

In at least one embodiment, first and second fluid circulation paths 502 and 504 are connected to waste line 588. When valve 590 is opened, IC media may flow through waste line 588 and to waste or outlet bag 586. Likewise, when valve 582 is opened, EC media may flow through waste line 588 to waste or outlet bag 586.

In embodiments, cells may be harvested via cell harvest path 596. Here, cells from hollow fiber bioreactor 501 may be harvested by pumping the IC media containing the cells through cell harvest path 596 and valve 598 to cell harvest bag 599.

Various components of the CES 500 may be contained or housed within a machine or housing, such as cell expansion machine 202 (FIGS. 2 and 3), wherein the machine maintains cells and media at a predetermined temperature.

Turning to FIG. 6, a schematic of another embodiment of a cell expansion system 600 is shown. CES 600 includes a first fluid circulation path 602 (also referred to as the "intracapillary loop" or "IC loop") and second fluid circulation path 604 (also referred to as the "extracapillary loop" or "EC loop"). First fluid flow path 606 may be fluidly associated with hollow fiber bioreactor 601 to form first fluid circulation path 602. Fluid flows into hollow fiber bioreactor 601 through IC inlet port 601A, through hollow fibers in hollow fiber bioreactor 601, and exits via IC outlet port 601B. Pressure sensor 610 measures the pressure of media leaving hollow fiber bioreactor 601. In addition to pressure, sensor 610 may, in embodiments, also be a temperature sensor that detects the media pressure and temperature during operation.

Media flows through IC circulation pump 612 which may be used to control the rate of media flow or rate of circulation. IC circulation pump 612 may pump the fluid in a first direction (e.g. counter clockwise) or second direction opposite the first direction (e.g., clockwise). Exit port 601B may be used as an inlet in the reverse direction. Media entering the IC loop may flow through valve 614. As those skilled in the art will appreciate, additional valves and/or other devices may be placed at various locations to isolate and/or measure characteristics of the media along portions of the fluid paths. Samples of media may be obtained from sample coil 618 during operation. Media then returns to IC inlet port 601A to complete fluid circulation path 602.

Cells grown/expanded in hollow fiber bioreactor 601 may be flushed out of hollow fiber bioreactor 601 into harvest bag 699 through valve 698 and line 697. Alternatively, when valve 698 is closed, the cells may be redistributed within hollow fiber bioreactor 601 for further growth. It is to be understood that the schematic shown represents one possible configuration for various elements of the CES 600, and modifications to the schematic shown are within the scope of the one or more present embodiments.

Fluid in second fluid circulation path 604 enters hollow fiber bioreactor 601 via EC inlet port 601C and leaves hollow fiber bioreactor 601 via EC outlet port 601D. Media in the EC loop may be in contact with the outside of the hollow fibers in the hollow fiber bioreactor 601, thereby allowing diffusion of small molecules into and out of the hollow fibers that may be within chamber 601, according to an embodiment.

Pressure/temperature sensor 624 disposed in the second fluid circulation path 604 allows the pressure and temperature of media to be measured before the media enters the EC space of the hollow fiber bioreactor 601. Sensor 626 allows the pressure and/or temperature of media in the second fluid circulation path 604 to be measured after it leaves the hollow fiber bioreactor 601. With regard to the EC loop, samples of media may be obtained from sample port 630 or a sample coil during operation.

After leaving EC outlet port 601D of hollow fiber bioreactor 601, fluid in second fluid circulation path 604 passes through EC circulation pump 628 to oxygenator or gas transfer module 632. EC circulation pump 628 may also pump the fluid in opposing directions, according to embodiments. Second fluid flow path 622 may be fluidly associated with oxygenator or gas transfer module 632 via an inlet port 632A and an outlet port 632B of oxygenator or gas transfer module 632. In operation, fluid media flows into oxygenator or gas transfer module 632 via inlet port 632A, and exits oxygenator or gas transfer module 632 via outlet port 632B. Oxygenator or gas transfer module 632 adds oxygen to and removes bubbles from media in the CES 600.

In various embodiments, media in second fluid circulation path 604 may be in equilibrium with gas entering oxygenator or gas transfer module 632. The oxygenator or gas transfer module 632 may be any appropriately sized device useful for oxygenation or gas transfer. Air or gas flows into oxygenator or gas transfer module 632 via filter 638 and out of oxygenator or gas transfer device 632 through filter 640. Filters 638 and 640 reduce or prevent contamination of oxygenator or gas transfer module 632 and associated media. Air or gas purged from the CES 600 during portions of a priming sequence may vent to the atmosphere via the oxygenator or gas transfer module 632.

In the configuration depicted for CES 600, fluid media in first fluid circulation path 602 and second fluid circulation path 604 flows through hollow fiber bioreactor 601 in the same direction (a co-current configuration). The CES 600 may also be configured to flow in a counter-current configuration.

In accordance with at least one embodiment, media, including cells (from a source such as a cell container, e.g. a bag) may be attached at attachment point 662, and fluid media from a media source may be attached at attachment point 646. The cells and media may be introduced into first fluid circulation path 602 via first fluid flow path 606. Attachment point 662 may be fluidly associated with the first fluid flow path 606 via valve 664, and attachment point 646 may be fluidly associated with the first fluid flow path 606 via valve 650. A reagent source may be fluidly connected to point 644 and be associated with fluid inlet path 642 via valve 648, or second fluid inlet path 674 via valves 648 and 672.

Air removal chamber (ARC) 656 may be fluidly associated with first circulation path 602. The air removal chamber 656 may include one or more sensors including an upper sensor and lower sensor to detect air, a lack of fluid, and/or a gas/fluid interface, e.g., an air/fluid interface, at certain measuring positions within the air removal chamber 656. For example, ultrasonic sensors may be used near the bottom and/or near the top of the air removal chamber 656 to detect air, fluid, and/or an air/fluid interface at these locations. Embodiments provide for the use of numerous other types of sensors without departing from the scope of the present disclosure. For example, optical sensors may be used in accordance with embodiments of the present disclosure. Air or gas purged from the CES 600 during portions of a priming sequence or other protocol(s) may vent to the atmosphere out air valve 660 via line 658 that may be fluidly associated with air removal chamber 656.

An EC media source may be attached to EC media attachment point 668 and a wash solution source may be attached to wash solution attachment point 666, to add EC media and/or wash solution to either the first or second fluid flow path. Attachment point 666 may be fluidly associated with valve 670 that may be fluidly associated with first fluid circulation path 602 via valve 672 and first fluid inlet path 642. Alternatively, attachment point 666 may be fluidly associated with second fluid circulation path 604 via second fluid inlet path 674 and second fluid flow path 684 by opening valve 670 and closing valve 672. Likewise, attachment point 668 may be fluidly associated with valve 676 that may be fluidly associated with first fluid circulation path 602 via first fluid inlet path 642 and valve 672. Alternatively, fluid container 668 may be fluidly associated with second fluid inlet path 674 by opening valve 676 and closing valve distribution 672.

In the IC loop, fluid may be initially advanced by the IC inlet pump 654. In the EC loop, fluid may be initially advanced by the EC inlet pump 678. An air detector 680, such as an ultrasonic sensor, may also be associated with the EC inlet path 684.

In at least one embodiment, first and second fluid circulation paths 602 and 604 are connected to waste line 688. When valve 690 is opened, IC media may flow through waste line 688 and to waste or outlet bag 686. Likewise, when valve 692 is opened, EC media may flow to waste or outlet bag 686.

After cells have been grown in hollow fiber bioreactor 601, they may be harvested via cell harvest path 697. Here, cells from hollow fiber bioreactor 601 may be harvested by pumping the IC media containing the cells through cell harvest path 697, with valve 698 open, into cell harvest bag 699.

Various components of the CES 600 may be contained or housed within a machine or housing, such as cell expansion machine 202 (FIGS. 2 and 3), wherein the machine maintains cells and media at a predetermined temperature. It is further noted that, in embodiments, components of CES 600 and CES 500 (FIG. 5) may be combined. In other embodiments, a CES may include fewer or additional components than those shown in FIGS. 5 and 6 and still be within the scope of the present disclosure. In embodiments, portions of CES 500 and 600 may be implemented by one or more features of the QUANTUM^{®} Cell Expansion System (CES), manufactured by Terumo BCT, Inc. of Lakewood, CO.

In one specific embodiment of using CES 600, hematopoietic stem cells (HSC's), e.g., CD34+ cells, may be expanded in an embodiment of CES 600. In this embodiment, HSC's (including CD34+ cells), which may be collected using a leukapheresis process or a manual process (e.g., umbilical cords), may be introduced into the bioreactor 601. The HSC's (including CD34+ cells) may be introduced into the bioreactor 601 through path 602.

In some embodiments, the HSC's (including CD34+ cells) may be subjected to a selection process (e.g., a purification process) before introduction into bioreactor 601. The process may involve the use of a centrifuge, purification column, magnetic selection, chemical selection, etc. Some examples of cell selection/ purification procedures include use of isolation columns from, for example, Miltenyi Biotec of Bergisch Gladbach, Germany. In one example, cord blood is first subjected to a cell selection process that selects for HSC's (including CD34+ cells) before the cells are introduced into the bioreactor 601. Other examples may utilize apheresis machines to deplete other cells that may be included with the HSC's (including CD34+ cells) when originally collected. For example, the HSC's may be sourced from cord blood, bone marrow, or peripheral blood. After initial collection, but before being introduced into the bioreactor 601, a volume of HSC's including CD34+ cells may be processed to deplete red blood cells, specific leukocytes, granulocytes, and/or other cells from the volume. These are merely some examples, and embodiments of the present invention are not limited thereto.

In other embodiments, the HSC's (including CD34+ cells) may be added directly to the bioreactor 601 after collection without any additional purification. For example, cord blood (with HSC's) may be added to the bioreactor. In addition to a number of proteins and other bioactive molecules, the cord blood may include HSC's (including CD34+ cells), red blood cells, platelets, granulocytes, and/or leukocytes.

It is noted that in some embodiments, the HSC's may be added to bioreactor 601, after a priming step. As may be appreciated, the cells being expanded may not be adherent and therefore it may not be required that they adhere to the hollow fiber walls of bioreactor 601 for expansion/proliferation. In these embodiments, the HSC's (including CD34+ cells) (purified or unpurified) may be introduced into the bioreactor 601 after a priming step and without a bioreactor coating step. If the cells were adherent cells, a coating step may be performed after the priming step and before introduction of the HSC's.

Once in the bioreactor 601, the cells may be exposed to growth factors, activators, hormones, reagents, proteins, and/or other bioactive molecules that may aid in the expansion of the cells. In one example, a co-culture cell line may have been previously grown/introduced, in the bioreactor 601, to optimize the conditions for growing the HSC's (including CD34+ cells). In one specific embodiment, human mesenchymal stem cells (hMSC's) may be co-cultured with the HSC's (including CD34+ cells) to promote growth of CD34+ cells. Without being bound by theory, it is believed that MSC's may emit factors (e.g., SDF-1 factors) that interact with HSC's (e.g., CD34+ cells) and promote proliferation of these cells. In some embodiments, use of the co-cultured hMSC's may involve a growing process that is performed initially, under conditions optimized for proliferating the hMSC's, before the HSC's (including CD34+ cells) are introduced into the bioreactor 601. The hMSC's may be derived in embodiments from bone marrow, peripheral blood, cord cells, adipose tissue, and/or molar tissue.

In addition to co-culture cells, a supplement including one or more growth factors, activators, hormones, reagents, proteins, and/or other bioactive molecules may be added to bioreactor 601 to grow and expand the HSC's. The supplement may be added as a single volume addition or over a period of time (e.g., continuously, intermittently, or on a regular schedule). In one embodiment, a combination of cytokines and/or other proteins, e.g., recombinant cytokines, hormones, etc., may be included as part of the supplement. As one example, a supplement may include one or more of: recombinant human Flt3 ligand (rhFlt-3L), recombinant human stem cell factor (rhSCF), recombinant human thrombopoietin (rhTPO), recombinant human (rh) Glial-derived neurotrophic factors and/or combinations thereof. One example of a supplement that may be used with embodiments is STEMCELL2MAX^{™} supplement (stemcell2MAX, Cantanhede, Portugal).

It is noted that in some embodiments, the combination of factors may be included in the media in which the cells are suspended. For example, the HSC's may be suspended in media and introduced into the bioreactor in the media. In embodiments, the media may include a combination of growth factors that aid in proliferation of the HSC's.

After the cells have been introduced into the bioreactor with the supplement, co-culture cells, and/or other material for expanding the cells, the cells are allowed to expand in bioreactor 601. During the expansion, there may be a number of materials that may be added or removed from bioreactor 601. As one example, additional proteins (e.g., cytokines) may be may be added to bioreactor 601. In some embodiments, more than one protein or other bioactive agent may be used. The additional material may be added individually, at the same time, at different times, or may be combined and added in combination.

It is noted that some embodiments may provide for adding material more directly into the bioreactor 601, such as through port 618. In other embodiments, however, the materials may be added in a location, e.g., through path 606, so that the materials may be perfused more slowly into bioreactor 601.

In addition to materials for aiding in growing the HSC's (including CD34+ cells), the HSC's may also be fed, such as by addition of a media that may include a number of nutrients. In some embodiments, the media may be commercially available media that may include serum. In other embodiments, the media may be serum free and include other additives. The media may be modified by the addition of other materials, some non-limiting examples including salts, serum, proteins, reagents, bioactive molecules, nutrients, etc. One example of media that may be used to feed the HSC's (including CD34+ cells) includes CELLGRO^{®} serum free media (CellGenix, Freiburg, Germany).

In some embodiments, while the co-culture cells are located in the IC space, feeding may occur in the EC space. Feeding through the EC space may, in embodiments, reduce the amount of force that may be felt by the cells from circulating fluid in the IC space. Circulation of media in the EC space may, in embodiments, provide sufficient nutrients for the expansion of the HSC's (including CD34+ cells).

As part of the expansion of the HSC's (including CD34+ cells), other conditions such as temperature, pH, oxygen concentration, carbon dioxide concentration, waste concentration, metabolite concentration etc. may also be controlled in bioreactor 601. In some embodiments, the flow rates of the EC side, e.g., path 604 may be used to control various parameters. For example, if it is desired to reduce waste or metabolite concentrations on the IC side, where the cells are growing, flow rate on the EC side may be increased to ensure that the waste and/or metabolites are removed from the IC side by migration through the hollow fibers from the IC side to the EC side.

After the CD34+ cells have been expanded, the cells may be removed from the bioreactor 601. The CD34+ cells may be collected in container 699. In embodiments, the collected CD34+ cells may be administered to a patient to reestablish hematopoiesis. Some non-limiting examples including patients undergoing treatment for various cancers, e.g., leukemia, myelodysplasia, non-Hodgkin lymphoma, etc., which may effect hematopoiesis. The cells may be administered with other compounds or molecules.

In some embodiments, use of CES 600 may provide advantages in growing HSC's (including CD34+ cells) over conventional processes. For example, the use of hollow fibers allows close cell to cell communication, which may enhance the growth of the CD34+ cells to start and continue to proliferate. Also, the use of a hollow fiber bioreactor, such as bioreactor 601, may provide a large surface area for cell growth, which may yield a higher concentration or higher volume of CD34+ cells.

Further, the conditions in bioreactor 601 may be controlled using a number of different components of the CES 600, including IC flow rates and EC flow rates. Also, CES 600 provides various locations for the addition of materials, which allows more direct, or indirect, e.g., perfusion, of cytokines into bioreactor 601.

Additionally, CES 600 provides a closed system. That is, the steps for growing the CD34+ cells may be performed without direct exposure to the ambient environment, which may contaminate the cells, or be contaminated by the cells or materials used in growing the cells. It is also believed that some embodiments may provide for using a smaller starting concentration of CD34+ cells for expansion, compared to other methods/systems. In these embodiments, CD34+ cells may also be expanded to yield larger amounts than from other methods/systems. It is also believed that some embodiments may provide for shortening the time for growing an effective dose of CD34+ cells.

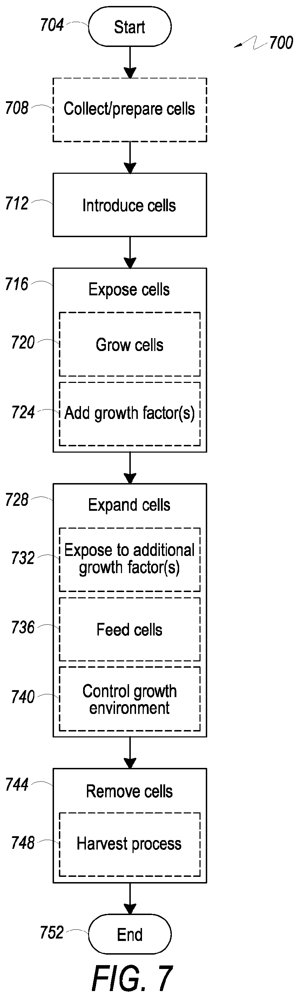

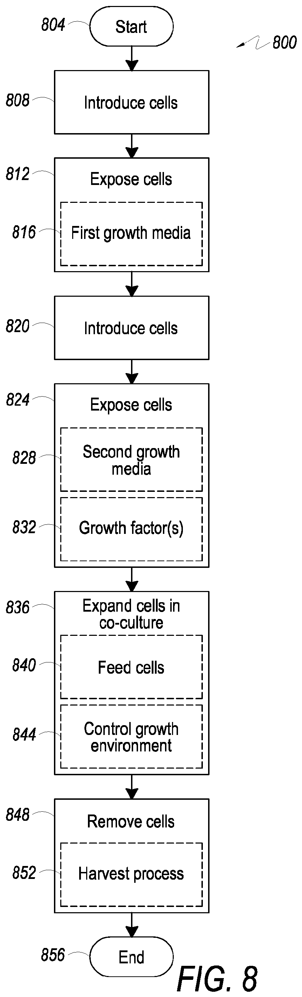

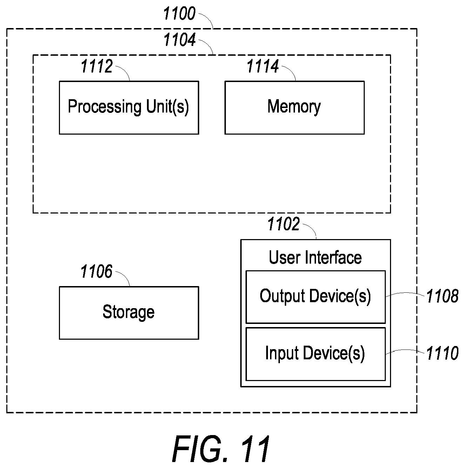

FIGS. 7-9 illustrate flows 700, 800, and 900 that may be performed to grow cells, e.g., HSC. Although specific devices may be described below for performing steps in flows 700, 800, and 900, embodiments are not limited thereto. For example, some steps may be described as performed by parts of a cell expansion system (e.g., CES's 500 or 600) or a processor (1100 (FIG. 11)), which may execute steps based on software provided as processor executable instructions. This is done merely for illustrative purposes, and flows 700, 800, and 900 are not limited to being performed by any specific device.

Flow 700 (an example useful for understanding the invention) starts at step 704 and proceeds to optional step 708 where cells (e.g., HSC's including CD34+ cells) may be collected/prepared. As one example, step 708 may involve an apheresis process, e.g., a leukapheresis process. In one specific example, an apheresis process is performed as part of step 708. Devices capable of collecting the cells include in an apheresis process include the SPECTRA OPTIA^{®} apheresis system, COBE^{®} spectra apheresis system, and the TRIMA ACCEL^{®} automated blood collection system, all manufactured by Terumo BCT, of Lakewood, Colorado.

In other examples, optional step 708 may involve thawing or otherwise preparing cells (e.g., from cord blood). In some examples, the preparation of the cord blood cells may involve processing the cord blood cells through a selection process that selects CD34+ cells. The process may be either a positive or a negative selection process. The process may involve the use of purification columns, magnetic columns, functionalized magnetic beads, reagents, or other materials that separate CD34+ cells from other cells. As one example, the selection process may involve the use of magnetic beads, functionalized with antigens, and devices such as the CLINIMACS PRODIGY^{®} system (Miltenyi Biotec, Bergisch Gladbach, Germany) that utilizes a magnetic column to complete the separation.

In other examples, apheresis machines may be used to deplete other cells that may be included with the cells when originally collected. For example, the cells may be sourced from cord blood, bone marrow, or peripheral blood. After initial collection, but before being introduced into a bioreactor, a volume of target cells may be processed to deplete red blood cells, specific leukocytes, granulocytes, and/or other cells from the volume before introduction into a bioreactor. This is merely one example and examples may utilize other materials and systems to effect the separation.

Flow 700 passes from step 708 to step 712, where cells may be introduced into a cell expansion system, in particular, a bioreactor of a cell expansion system. As noted above, in some examples, flow 700 may begin at step 712. In examples, the bioreactor may be a hollow fiber bioreactor such as bioreactor 100 (FIG. 1). In these examples, step 712 may involve flowing cells into one or more individual hollow fibers. Step 712 may involve the use of a processor, pumps, valves, fluid conduit, etc. to introduce cells into a bioreactor. In one example, step 712 may involve opening valves (e.g., 564, 514, 664, and/or 614) and activating pumps (e.g., 554 and 654).

After step 712, flow 700 passes to step 716 where the cells are exposed to one or more growth conditions. As may be appreciated, certain cell types require specific growth conditions to expand. The growth conditions may for example include exposure to certain proteins. One example of this type of cell includes CD34+ cells. Step 716 provides for exposing cells to any necessary growth factors, proteins, reagents, nutrients, etc. that may promote cell expansion and growth.

Step 716 may involve a number of steps that may be performed as part of step 716, or precede step 716. For example, in some examples, step 720 may be performed to grow co-culture cells that may generate material that promotes growth of the target cell line. In one example, step 720 may be performed prior to step 716, or even prior to step 708. In one specific example, human mesenchymal stem cells (hMSC's) may be grown in the bioreactor. In these examples, step 720 may involve a number of steps (e.g., collecting hMSC's from bone marrow, peripheral blood, cord cells, adipose tissue, and/or molar tissue) that result in co-culture cells being present in the bioreactor during step 716. As may be appreciated, these steps may be performed prior to step 712.

With respect to growing the co-culture cells, several steps for conditioning the bioreactor for growing the co-culture cells may be performed. In examples, the steps may include priming the bioreactor, coating the bioreactor with materials that promote the attachment of the co-culture cells (e.g., when the co-culture cells are adherent cells), washing to remove materials prior to introduction of the co-culture cells into the bioreactor, attaching the co-culture cells, and expanding the co-culture cells.

In other examples, step 716 may involve step 724, where growth factors may be added to the bioreactor. As may be appreciated, in some examples, one or more growth factors, e.g., cytokines, may be added to promote the proliferation of cells. As one example, when growing CD34+ cells, the growth factor(s) may include one or more of: recombinant human Flt3 ligand (rhFlt-3L), recombinant human stem cell factor (rhSCF), recombinant human thrombopoietin (rhTPO), recombinant human (rh) Glial-derived neurotrophic factors, and combinations thereof.

In one example, at least a portion of the growth factors may be introduced with the cells at step 712. For example, the media in which the cells are in when introduced into the bioreactor may be conditioned with a combination of growth factors including one or more of recombinant human Flt3 ligand (rhFlt-3L), recombinant human stem cell factor (rhSCF), recombinant human thrombopoietin (rhTPO), recombinant human (rh) Glial-derived neurotrophic factors, and combinations thereof. In these examples, step 716, and optional step 724, may involve supplementing growth factors that may have already been added to the bioreactor with the cells.

After step 716, flow may pass to step 728, where cells are expanded, i.e., proliferated. Step 728 may involve a number of sub-steps. For example, at sub-step 732, the cells may be exposed to additional growth factors, proteins, bioactive molecules, etc. The exposure may provide for the cells to continue to proliferate.

In examples, step 728 may occur during a period of time of several hours, several days, or even several weeks. During this period of time, the various sub-steps (e.g., 732, 736, and/or 740) may be performed at various times during this period. For example, during the period, additional substances such as growth factors may be perfused or directly injected into the bioreactor to supplement and continue to expose the cells and promote proliferation.

Step 728 may also involve a feeding cells step 736. Step 736 may involve adding various nutrients, including glucose, phosphates, salts, etc. Step 736 may in examples involve sensing a concentration of a nutrient and in response to a relatively low concentration, adding nutrient(s) to the bioreactor. Step 736 may involve the use of a processor, pumps, valves, fluid conduit, etc. to add nutrients to feed the cells. Alternatively, the additions of nutrients, such as glucose may be added based on a predetermined schedule.

In some embodiments, step 728 may involve circulating media in the EC space. The media may include nutrients necessary for expansion of the CD34+ cells. Feeding through the EC space may, in examples, reduce the amount of force that may be felt by the cells if media is circulated in the IC space. Circulation of media in the EC space may, in examples, provide sufficient nutrients (e.g., glucose) for the expansion of the HSC's (including CD34+ cells) through diffusion of the nutrients through hollow fibers. In other examples, step 728 may involve circulating media, with nutrients (e.g., glucose), in the IC space for more direct feeding.

Step 740 may involve controlling the growth environment of the bioreactor. As may be appreciated, in addition to nutrients, the environment for optimizing growth of cells may involve a number of different parameters. For example, the temperature, pH, oxygen concentration, carbon dioxide concentration, waste concentration, metabolite concentration etc. may be monitored and controlled as part of step 740. In one specific example, a flow rate of a fluid flowing through an extracapillary space (EC) side of a bioreactor may be used in controlling the pH, oxygen concentration, carbon dioxide concentration, waste concentration, metabolite concentration etc. in the intracapillary space where the cells are expanding.

Flow 700 proceeds from step 740 to step 744 where cells are removed from the bioreactor. Step 744 may involve a number of sub-steps. For example, a harvest process 748, which itself includes a number of steps, may be performed as part of step 744. In examples, step 748 may involve changing circulation rates on the intracapillary space (IC) and extra capillary space (EC) sides of the bioreactor. In other examples, step 744 may involve circulating various materials to ensure that any cells that may have attached themselves to the inside surface of fibers are released and removed from the bioreactor. As one example, a protease may be added to break down proteins, such as glycoproteins that may aid in binding of the cells to the fibers.

In one example, as part of a harvest process 748, fluid within the bioreactor (e.g., intracapillary space) may be circulated at a relatively high rate. The circulation of the fluid may promote suspension of the cell expanded at step 728. The circulation may occur for a predetermined period of time to ensure that the expanded cells are suspended in the fluid and as many as possible may be recovered from the bioreactor. Flow 700 ends at step 752.

Flow 800 may be performed in embodiments to grow cells, such as CD34+ cells in co-culture. In embodiments, the co-culture may improve growth of the CD34+ cells. Flow 800 starts at step 804 and proceeds to step 808 where first cells (e.g., human mesenchymal stem cells (hMSC's)) may be introduced into a bioreactor. In embodiments, the bioreactor (e.g., bioreactor 100 (FIG. 1)) may be a hollow fiber bioreactor that includes a number of hollow fibers (e.g., hollow fibers 116 (FIG. 1)).

In embodiments, hollow fibers in the hollow fiber bioreactor are conditioned prior to step 808. In order to allow the hMSC's to adhere to the interior wall of the hollow fibers, the hollow fibers are coated with fibronectin. The coating process may involve a number of substeps.

The cells introduced at step 808 are introduced into the intracapillary space, e.g., in the interior of the hollow fibers, of the hollow fiber bioreactor. Step 808 may involve the use of a processor (1100, (FIG. 11)), pumps, valves, fluid conduit, etc. to introduce cells into a bioreactor. In one embodiment, step 808 may involve opening valves (e.g., 564, 514, 664, and/or 614) and activating pumps (e.g., 554 and 654).

Step 808 may involve the use of a processor (1100, (FIG. 11)), pumps, valves, fluid conduit, etc. to introduce cells into a bioreactor. In one embodiment, step 808 may involve opening valves (e.g., 570, 576, 670, and/or 676) and activating pumps (e.g., 578 and/or 678).

Flow passes from step 808 to step 812, where the first cells are exposed to first growth conditions. The first growth conditions may be optimized for growing the first cells introduced at step 808. Step 812 involves feeding the first cells with a first growth media (step 816) that includes nutrients for growing the first cells. The growth media may include one or more of various nutrients, including glucose, phosphates, salts, etc.

After a predetermined period of time (which may depend on a certain number of the first cells having been grown within the bioreactor), flow 800 passes to 820 where second cells (comprising CD34+) are introduced into the bioreactor. In embodiments, the second cells are introduced into an intracapillary space just like the first cells.

In some embodiments, prior to introducing second cells, a washout procedure may be performed to remove the previous fluid from the bioreactor. As may be appreciated, the fluid in the bioreactor may include nutrient tailored for growing the first cells. Embodiments may provide for flushing this fluid out of the bioreactor prior to, or as part of step 820, and introducing the second cells in to the bioreactor.

Step 820 may involve the use of a processor (1100, (FIG. 11)), pumps, valves, fluid conduit, etc. to introduce second cells into a bioreactor. In one embodiment, step 820 may involve opening valves (e.g., 564, 514, 664, and/or 614) and activating pumps (e.g., 554 and 654).

Flow passes from step 820 to step 824, where the second cells are exposed to second growth conditions. The second growth conditions may be optimized for growing the second cells introduced at step 820. For example, step 820 may involve feeding the second cells with a second growth media (at optional step 828) that includes nutrients for growing the second cells. The second media may include one or more of various nutrients, including glucose, phosphates, salts, etc.

Additionally, step 820 involves the step of 832 adding growth factors that promote growth of the second cells, (comprising CD34+ cells). In embodiments, the growth factors, e.g., cytokines, may be added to promote the proliferation of cells. The growth factors include a combination of: recombinant human Flt3 ligand (rhFlt-3L), recombinant human stem cell factor (rhSCF), recombinant human thrombopoietin (rhTPO), recombinant human (rh) Glial-derived neurotrophic factors. In embodiments, the growth factors may be added to the intracapillary space of the hollow fiber bioreactor.

In embodiments, at least a portion of the growth factors may be introduced with the cells at step 820. For example, the media in which the cells are in when introduced into the bioreactor may be conditioned with a combination of growth factors including one or more of recombinant human Flt3 ligand (rhFlt-3L), recombinant human stem cell factor (rhSCF), recombinant human thrombopoietin (rhTPO), recombinant human (rh) Glial-derived neurotrophic factors, and combinations thereof. In these embodiments, step 820, and step 832, may involve supplementing growth factors that may have already been added to the bioreactor with the cells.

Flow passes from step 824 to step 836, where cells are expanded in co-culture. Step 836 may involve a number of optional sub-steps. For example, the cells may be fed at optional sub-step 840. Additional glucose or other nutrients may be provided to the cells as they expand in co-culture. At sub-step 844, other conditions may be controlled to expand the cells. For example, temperature, pH, gas concentrations, etc. may be monitored and changed in order to control the environment for expansion of the cells.

Flow 800 proceeds from step 836 to step 848 where cells are removed from the bioreactor. Step 848 may involve a number of sub-steps. For example, a harvest process 852, which itself includes a number of steps, may be performed as part of step 848. In embodiments, step 848 may involve changing circulation rates on the intracapillary space (IC) and extra capillary space (EC) sides of the bioreactor. In other embodiments, step 848 may involve circulating various materials to ensure that any cells that may have attached themselves to the inside surface of fibers are released and removed from the bioreactor. As one example, a protease may be added to break down proteins, such as glycoproteins (fibronectin, collagen) that may aid in binding of the cells to the fibers.

In one embodiment, as part of a harvest process 852, fluid within the bioreactor (e.g., intracapillary space) may be circulated at a relatively high rate. The circulation of the fluid may promote suspension of the cell expanded at step 836. The circulation may occur for a predetermined period of time to ensure that the expanded cells are suspended in the fluid and as many as possible may be recovered from the bioreactor. Flow 800 ends at step 856.

Flow 900 starts at step 904 and proceeds to step 908 where first cells (e.g., human mesenchymal stem cells (hMSC's)) may be grown in a static growth chamber. Step 908 may involve use of a flask or a gas permeable reactor, where fluid is not moved by pumps. Rather, the growth chamber remains stationary and fluid does not flow after being added to the chamber.

Flow 900 may then pass from step 908 to step 912 where second cells are grown statically in the static growth chamber. In embodiments, the second cells may be hematopoietic progenitor cells (e.g., CD34+ cells). After step 912, the second cells, which may have been expanded in the static growth chamber, may be removed from the growth chamber, for additional expansion in a dynamic cell expansion system (e.g., CES's 500 and/or 600) where fluid may be circulated (e.g., automatically) as the cells are expanded.

Flow 900 then passes to step 920, where first cells (e.g., hMSC's) are introduced into a hollow fiber bioreactor that includes a number of hollow fibers. The cells introduced at step 920 are introduced into the interior (lumen) of the hollow fibers.

In embodiments, hollow fibers in the hollow fiber bioreactor are conditioned prior to step 920. In order to allow the hMSC's to adhere to the interior wall of the hollow fibers, the hollow fibers are coated with fibronectin. The coating process may involve a number of substeps.

Step 920 may involve the use of a processor (1100, (FIG. 11)), pumps, valves, fluid conduit, etc. to introduce first cells into a bioreactor. In one embodiment, step 920 may involve opening valves (e.g., 564, 514, 664, and/or 614) and activating pumps (e.g., 554 and 654).

Step 920 may involve a number of sub-steps. In embodiments, as part of introducing cells into the hollow fiber bioreactor, the bioreactor may be rotated at sub-step 922. For example, in order to have the first cells attach to as much of the interior (lumen) of the hollow fibers as possible, the bioreactor may be rotated in a specific pattern.

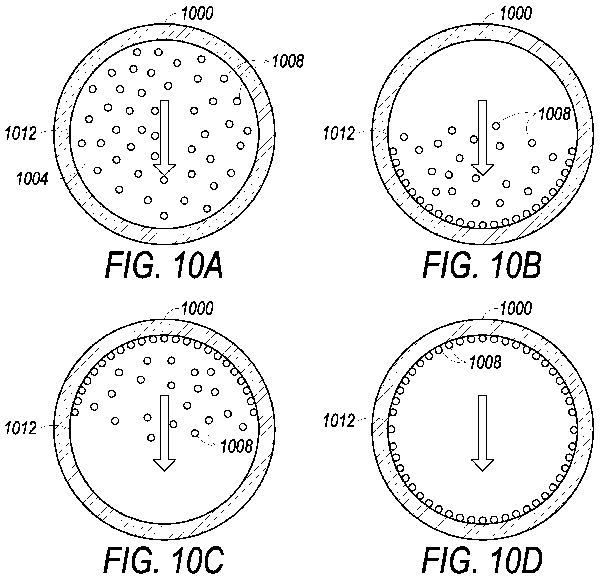

FIGS. 10A-D illustrate a cross-section of a hollow fiber 1000 (e.g., fibers 116 (FIG. 1)) during a process of introducing, and attaching, first cells to an interior of hollow fibers. In FIG. 10A, fluid 1004 with first cells 1008 is circulated around an intracapillary space of a hollow fiber membrane with a pump (see FIGS. 5 and 6 and description above). As shown in FIG. 10A, initially 1008 cells are distributed throughout the fluid.

The pump may then be stopped, which results in the first cells settling, and after a period of time, attaching onto a portion of the inside 1012 of the hollow fiber, as shown in FIG. 10B. In embodiments, the hollow fiber has previously been coated with a compound to promote adhesion of the cells to the hollow fiber inside wall. The lumen of the hollow fiber has been coated with fibronectin.

After the first cells 1008 have settled and attached, the bioreactor (and consequently fiber 1000) may be rotated 180 degrees. Examples of methods for rotating a bioreactor when loading cells, in order to distribute cells more evenly on the inside surface of hollow fibers, is described in at least U.S. Patent No. 9,617,506, issued April 11, 2017, entitled "EXPANDING CELLS IN A BIOREACTOR".

In some embodiments, after the rotation, the pump may be activated again to circulate the remaining, unattached, first cells 1008 in the intracapillary space of the hollow fiber membrane. In other embodiments, the pump may not be reactivated.

As shown in FIGS. 10C, a second portion of the first cells 1008 may then begin to settle and attach to a second portion of the inside 1012 of the hollow fiber 1000. After a period of time, a second portion of the first cells 1008 may attach as shown in FIG. 10D. In this way, the first cells 1008 (e.g., hMSC's) may be distributed more evenly around an entire inside surface 1012, e.g., both top and bottom of the inside of a hollow fiber.

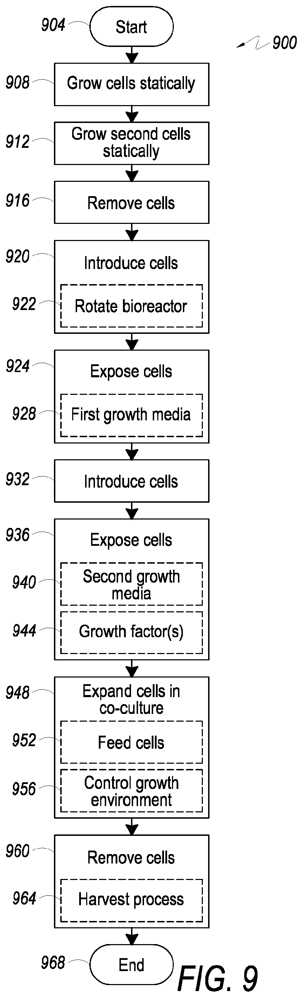

Referring again to FIG. 9, flow passes from step 920 to step 924, where the first cells are exposed to first growth conditions. The first growth conditions may be optimized for growing the first cells introduced at step 920. Step 924 involves feeding the first cells with a first growth media (step 928) that includes nutrients for growing the first cells. The growth media may include one or more of various nutrients, including glucose, phosphates, salts, etc.

After a predetermined period of time (which may depend on a certain number of the first cells having been grown within the bioreactor), flow 900 passes to 932 where second cells (CD34+) are introduced into the bioreactor. In embodiments, the second cells are introduced into an intracapillary space just like the first cells.

In some embodiments, prior to introducing second cells, a washout procedure may be performed to remove the previous fluid from the bioreactor. As may be appreciated, the fluid in the bioreactor may include nutrient tailored for growing the first cells. Embodiments may provide for flushing this fluid out of the bioreactor prior to, or as part of step 932, and introducing the second cells in to the bioreactor.

Step 932 may involve the use of a processor (1100, (FIG. 11)), pumps, valves, fluid conduit, etc. to introduce second cells into a bioreactor. In one embodiment, step 932 may involve opening valves (e.g., 564, 514, 664, and/or 614) and activating pumps (e.g., 554 and 654).

Flow passes from step 932 to step 936, where the second cells are exposed to second growth conditions. The second growth conditions may be optimized for growing the second cells introduced at step 932. For example, step 936 may involve feeding the second cells with a second growth media (at optional step 940) that includes nutrients for growing the second cells. The second media may include one or more of various nutrients, including glucose, phosphates, salts, etc.

Additionally, step 936 involves the step 944 of adding growth factors that promote growth of the second cells (comprising CD34+ cells). In embodiments, the growth factors, e.g., cytokines, may be added to promote the proliferation of cells. The growth factors include a combination of: recombinant human Flt3 ligand (rhFlt-3L), recombinant human stem cell factor (rhSCF), recombinant human thrombopoietin (rhTPO), recombinant human (rh) Glial-derived neurotrophic factors. In embodiments, the growth factors may be added to the intracapillary space of the hollow fiber bioreactor.

In one embodiment, at least a portion of the growth factors may be introduced with the cells at step 936. For example, the media in which the cells are in when introduced into the bioreactor may be conditioned with a combination of growth factors including one or more of recombinant human Flt3 ligand (rhFlt-3L), recombinant human stem cell factor (rhSCF), recombinant human thrombopoietin (rhTPO), recombinant human (rh) Glial-derived neurotrophic factors, and combinations thereof. In these embodiments, step 936, and step 944, may involve supplementing growth factors that may have already been added to the bioreactor with the cells.

Flow passes from step 936 to step 948, where cells are expanded in co-culture. Step 948 may involve a number of optional sub-steps. For example, the cells may be fed at optional sub-step 952. Additional glucose or other nutrients may be provided to the cells as they expand in co-culture. At sub-step 956, other conditions may be controlled to expand the cells. For example, temperature, pH, gas concentrations, etc. may be monitored and changed in order to control the environment for expansion of the cells.

Flow 900 proceeds from step 948 to step 960 where cells are removed from the bioreactor. Step 960 may involve a number of sub-steps. For example, a harvest process 964, which itself includes a number of steps, may be performed as part of step 960. In embodiments, step 964 may involve changing circulation rates on the intracapillary space (IC) and extra capillary space (EC) sides of the bioreactor. In other embodiments, step 964 may involve circulating various materials to ensure that any cells that may have attached themselves to the inside surface of fibers are released and removed from the bioreactor. As one example, a protease may be added to break down proteins, such as glycoproteins that may aid in binding of the cells to the fibers.

In one embodiment, as part of a harvest process 964, fluid within the bioreactor (e.g., intracapillary space) may be circulated at a relatively high rate. The circulation of the fluid may promote suspension of the cell expanded at step 948. The circulation may occur for a predetermined period of time to ensure that the expanded cells are suspended in the fluid and as many as possible may be recovered from the bioreactor. Flow 900 ends at step 968.

With respect to flows 700, 800, and 900 illustrated in FIGS. 7-9, the operational steps depicted are offered for purposes of illustration and may be rearranged, combined into other steps (e.g., add steps 908 and/or 912 into flows 700 and/or 800), used in parallel with other steps, etc., according to embodiments of the present disclosure. Fewer or additional steps may be used in embodiments without departing from the scope of the present disclosure. Also, steps (and any sub-steps) may be performed automatically in some embodiments, such as by a processor (1100 (FIG. 11)) executing pre-programmed tasks stored in memory, in which such steps are provided merely for illustrative purposes.

Also, it is noted that although flows 700, 800, and 900 have been described above with various steps in particular order, the present invention is not limited thereto. In other embodiments, the various steps and sub-steps may be performed in a different order, in parallel, partially in the order shown in FIGS. 7-9, and/or in sequence as shown in FIGS. 7-9. Also, the description above indicating that the step or sub-steps are performed by particular features or structures is not intended to limit the present invention. Rather, the description is provided merely for illustrative purposes. Other structures or features not described above may be used in other embodiments to perform one or more of the steps of flows 700, 800, and 900. Furthermore, flows 700, 800, and 900 may include some optional steps. However, those steps above that are not indicated as optional should not be considered as essential to the invention, but may be performed in some embodiments of the present invention and not in others.

FIG. 11 illustrates example components of a computing system 1100 upon which embodiments of the present disclosure may be implemented. Computing system 1100 may be used in embodiments, for example, where a cell expansion system uses a processor to execute tasks, such as custom tasks or pre-programmed tasks performed as part of processes, such as the process illustrated by flows 700, 800, and 900 and described above.

The computing system 1100 may include a user interface 1102, a processing system 1104, and/or storage 1106. The user interface 1102 may include output device(s) 1108, and/or input device(s) 1110 as understood by a person of skill in the art. Output device(s) 1108 may include one or more touch screens, in which the touch screen may comprise a display area for providing one or more application windows. The touch screen may also be an input device 1110 that may receive and/or capture physical touch events from a user or operator, for example. The touch screen may comprise a liquid crystal display (LCD) having a capacitance structure that allows the processing system 1104 to deduce the location(s) of touch event(s), as understood by those of skill in the art. The processing system 1104 may then map the location of touch events to user interface (UI) elements rendered in predetermined locations of an application window. The touch screen may also receive touch events through one or more other electronic structures, according to embodiments. Other output devices 1108 may include a printer, speaker, etc. Other input devices 1110 may include a keyboard, other touch input devices, mouse, voice input device, etc., as understood by a person of skill in the art.

Processing system 1104 may include a processing unit 1112 and/or a memory 1114, according to embodiments of the present disclosure. The processing unit 1112 may be a general purpose processor operable to execute instructions stored in memory 1114. Processing unit 1112 may include a single processor or multiple processors, according to embodiments. Further, in embodiments, each processor may be a multi-core processor having one or more cores to read and execute separate instructions. The processors may include general purpose processors, application specific integrated circuits (ASICs), field programmable gate arrays (FPGAs), other integrated circuits, etc., as understood by a person of skill in the art.

The memory 1114 may include any short-term or long-term storage for data and/or processor executable instructions, according to embodiments. The memory 1114 may include, for example, Random Access Memory (RAM), Read-Only Memory (ROM), or Electrically Erasable Programmable Read-Only Memory (EEPROM), as understood by a person of skill in the art. Other storage media may include, for example, CD-ROM, tape, digital versatile disks (DVD) or other optical storage, tape, magnetic disk storage, magnetic tape, other magnetic storage devices, etc., as understood by a person of skill in the art.

Storage 1106 may be any long-term data storage device or component. Storage 1106 may include one or more of the systems described in conjunction with the memory 1114, according to embodiments. The storage 1106 may be permanent or removable. In embodiments, storage 806 stores data generated or provided by the processing system 104.

### EXAMPLES

Some examples that may implement aspects of the embodiments are provided below. Although specific features may be described in these examples, they are provided merely for illustrative and descriptive purposes. The present invention is not limited to the examples provided below.

### EXAMPLE 1

Initial expansion of unselected cord blood-derived (CB) HSCs, CD34+ cells from three (3) donors are acquired from AllCells^{®} (Alameda, CA) by positive immunomagnetic selection of cord blood and grown in co-culture with bone marrow-derived hMSCs under static conditions (5%CO₂ & 37.0°C) with serum-free CellGenix (Freiburg, Germany) CellGro^{®} GMP SCGM with the addition of StemCell2MAX^{™} supplement (rhFlt-3L, rhSCF, rhTPO, rhGlial-derived neurotrophic factors) at a 1:100 concentration to develop the inoculum for a hollow fiber cell expansion system, e.g., the Quantum^{®} Cell Expansion System (CES). Each of the Quantum CES bioreactors is coated with five (5) mg of human fibronectin overnight and seeded with unmatched hMSCs at 3.0x10³/cm² five (5) days in advance of HSC introduction. The bioreactors are seeded with CB-derived CD34+ cells in CellGro^{®} GMP SCGM + StemCell2MAX at a cell density of 3.0x10⁴/mL for a total seeding of 5.7x10⁶ cells and subsequently expanded for 6.6 days using a mixed gas (5%CO₂, 20%O₂, balance N₂) at 37.0°C in co-culture. Cells are seeded to the bioreactor, expanded, and harvested using automated tasks on the Quantum^{®} CES. Specifically, cells are grown in the intracapillary loop of the bioreactor and base media additions are made through the extracapillary loop of the system in order to enhance hMSC-CB CD34+ cell interactions. Metabolites are quantified daily with Abbott i-STAT^{®} analyzers. Cells are counted with a Beckman Coulter Vi-CELL^{™} XR Cell Viability Analyzer using a diameter size range of 5-50 µm. Harvested cells are stained for surface biomarkers with BD Mouse Anti-Human CD34-FITC, BD Mouse Anti-Human CD34-PE, BD Mouse Anti-Human CD38-APC, MB CD133/1-PE, and eBioscience Fixable Viability Dye eFluor^{®} 780 and analyzed by flow cytometry using a BD FACSCanto II equipped with BD FACSDiva software. Fluorescent microscopy images are captured with a Zeiss Axio Observer A1 microscope equipped with ZEN pro software. The Stem Cell Technologies Human CFU Methocult^{™} Assay is used to induce the differentiation of harvested CB-derived CD34+ cells.

**Table 1: CB-derived CD34⁺ Cell Seeding and Harvest Cell Numbers**

| **CES System** | | | | | | | |
|---|---|---|---|---|---|---|---|
| **Yield** | **Bioreactor** | **Bioreactor** | **Vi-CELL** | **Fold** | **Expansion** | | |
| **CES Run** | **Seeding** | **Harvest** | **Viability** | **Increase** | **Days** | **DS** | **DT (hrs)** |
| Donor 1 | 5.70x10⁶ | 7.95x10⁷ | 83.8% | 13.9 | 6.6 | 3.8 | 41.7 |
| Donor 2 | 5.70x10⁶ | 9.94x10⁷ | 85.1% | 17.4 | 6.6 | 4.1 | 38.4 |
| Donor 3 | 5.70x10⁶ | 8.89x10⁷ | 89.2% | 15.6 | 6.6 | 4.0 | 40.0 |
| ***Average*** | ***5.70x10⁶*** | ***8.93x10⁷*** | ***86.0%*** | ***15.7*** | ***6.6*** | ***4.0*** | ***40.0*** |

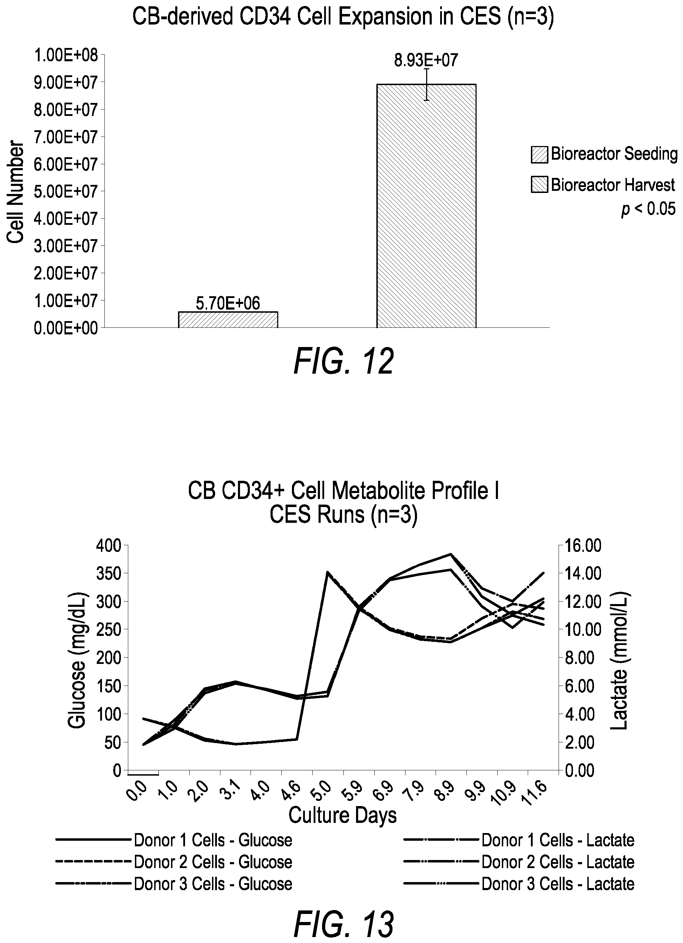

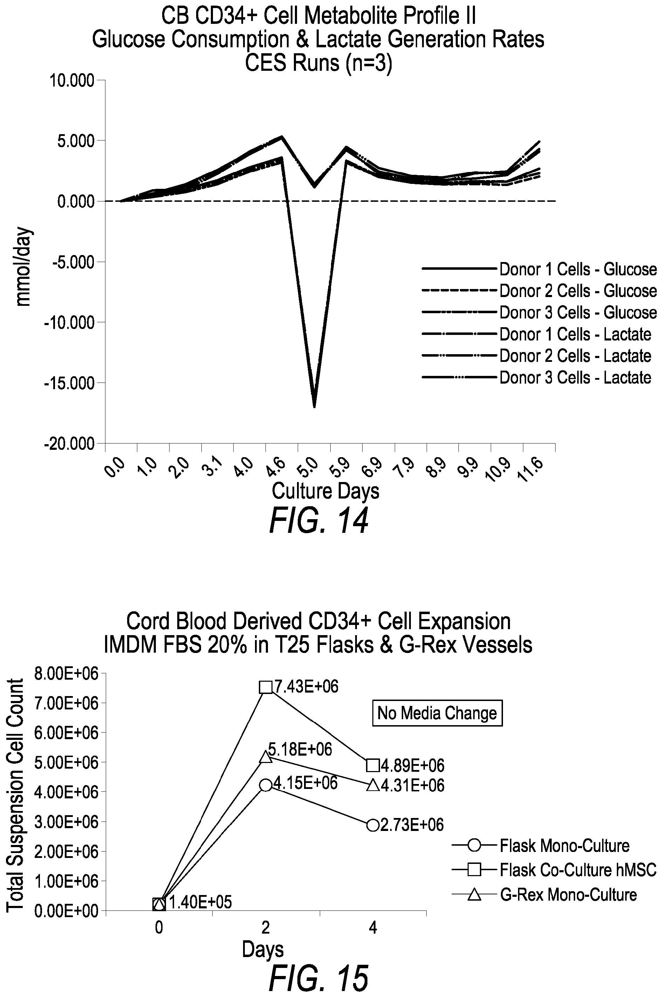

FIG. 12 illustrates a bar graph of some of the data shown above in Table 1, namely showing the average number of cells harvested from the various donors. FIG. 13 illustrates a graph showing metabolic profile for the various donors. FIG. 14 illustrates a graph showing metabolic rates for the various runs.

The results may suggest that the Quantum CES hollow fiber membrane bioreactor can support the expansion of CB-derived CD34+, CD38-CD133+ progenitor cells in co-culture using an automated CES. The growth factor supplement of rhFlt-3L, rhSCF, rhTPO, rhGlial-derived neurotrophic factors may support the co-culture of CB-derived CD34+ lineage cells. Expanded CB-derived CD34+ cells may demonstrate lineage differentiation in the presence of appropriate cytokines and supplements (SCF, GM-CSF, IL-3, GSF, EPO). Table 2 below provides a summary of flow cytometry results.

**Table 2: Summary Of Flow Cytometry Results**

| **Quantum Run** | **CD34⁺CD38⁻** | **CD34⁺CD38^{+/-}** | **CD34⁺CD133⁺** |
|---|---|---|---|
| Donor 1 | 4.3% | 4.9% | 1.0% |
| Donor 2 | 5.1% | 6.8% | 2.5% |
| Donor 3 | 4.3% | 7.3% | 0.8% |
| ***Average*** | ***4.6%*** | ***6.3%*** | ***1.4%*** |

### EXAMPLE 2

The culture of I-Mag selected human HSC CB CD34+ cells (frozen CB008F-2) obtained from AllCells using IMDM media supplemented with FBS or human AB serum in mono- or co-culture with human mesenchymal stem cells (hMSCs) to improve the media cost framework of CD34+ expansion is explored. Cell culture devices include the Wilson G-Rex 10 Gas Permaeable Membrane devices and conventional tissue culture polystyrene flasks. After the results of the first experiment, a decision is made to switch to a StemSpan SPEM II media containing modified IMDM (BSA, rh-insulin, transferrin) plus FBS or Human Serum AB as well as to increase the cell seeding density in order to improve cell growth and biomarker expression.

### Experiment 1

T25 TCPS Flasks are seeded with hMSC-P2T1 at 1,000 cells/cm² in Gibco alpha-MEM (Cat. No. 32561-037) Complete Medium (CM224), incubated at 37°C (5% CO₂), and grown to 80% confluency on Day-6 prior to seeding with thawed CB CD34+ cells. By comparison, no hMSCs are seeded into the Wilson G-Rex gas permeable cell culture device. CB CD34+ cells are seeded at a concentration of 2.0x10⁴/mL and grown with 7 mL of Gibco IMDM (Cat. No. 31980-030), 20% HyClone FBS (Cat. No. SH30070.03) or 18% Akron HS-AB (Cat. No. AK9340-0100), plus Gibco Penicillin/Streptomycin/Neomycin antibiotics (Cat. No. 15640-055) for four (4) days. The CD34+ cell seeding density is based on a recommendation to maintain the cell concentration below 3.0x10⁴/mL. Culture vessels are weighed to determine culture volumes and cell counts are captured with a calibrated Beckman Coulter Vi-Cell XR Cell Viability Analyzer on Days 0, 2, and 4.

### Experiment 2

Six (6) T25 TCPS Flasks are seeded with hMSC-P2T1 at 8.48x10³/cm² in Gibco alpha-MEM Complete Medium (CM231), incubated at 37°C (5% CO₂), and grown to 80% confluency on Day-3 prior to seeding with thawed (37°C) CB CD34+ cells (AllCells CB-CD34+, Cat. No. CB008F-2, ID No. CBP140129C). CB CD34+ cells (2.5 mL) are resuspended in 22.5 mL of StemSpan SFEM II media, centrifuged at 500xg for 7 minutes, resuspended in 10 mL of respective complete media, and counted with a calibrated Beckman Coulter Vi-Cell NR Cell Viability Analyzer. Each flask media is exchanged with 6 mL of sterile filtered (Corning 0.22 µm PES) StemSpan SFEM II Complete Media 20%FBS (HyClone Cat. No. SH30070.03) or 20% HS-AB (Innovative Research (Cat. IPLA-SERAB-OTC-16138), Gibco Pennicillin/Streptomycin/Neomycin Antibiotics (Cat. No. 15640-055). A total of four (4) T25 co-culture flasks are seeded with CB CD34+ cells at 3.00x10⁴/mL or 3.00x10⁵/mL into 6 mL per flask with StemSpan SFEM II media supplemented with either 20% FBS or 20% HS-AB, incubated at 37°C (5% CO₂) for four (4) days. Suspension cells from each flask are removed on Day-2, transferred to a 15 mL sterile centrifuge tube, centrifuged at 500xg for 7 minutes, resuspended in 6 mL of their respective Complete StemSpan SFEM II media, and returned to T25 co-culture flasks for incubation. On Day-4, images of suspension cells are captured by phase-contrast photomicroscopy (Olympus CKX41 with QCapture Pro 6.0 software), flasks are weighed to determine flask volume, and cells are counted as previously described. Aliquots (1.25x10⁵ cells) of the high cell seeding density are prepared for flow cytometry staining (BD Pharmingen FITC Mouse Anti-human CD34 Cat. 555821, BD Pharmingen FITC Mouse IgG1κ isotype control Cat. 555748, BD Pharmingen APC Mouse Anti-human CD38 Cat. 555462, BD Pharmingen Mouse IgG1κ Isotype Control Cat. 555751, BD Pharmingen PE Mouse Anti-human CD34 Cat. 555822, BD Pharmingen PE Mouse IgG1κ Isotype Control Cat. 555749, Miltenyi Biotec CD133/1-PE Cat. 180-080-801) using a modified version of the Flow Cytometry Prep Protocol. Flow data are acquired and analyzed without fixative using a BD FACSCanto^{®} II flow cytometer equipped with BD FACSDiva^{®} v6.1.3 software.

Experiment 1 may evaluate the mono- and co-culture of cord blood derived CD34+ cells at the low seeding density of 2.00x10⁴/mL in a total volume of 7mL using IMDM base media supplemented with 20% FBS or 18% HS. T25 tissue culture polystyrene (TCPS) flasks and gas permeable G-Rex-10 vessels are used as the primary static culture environment.

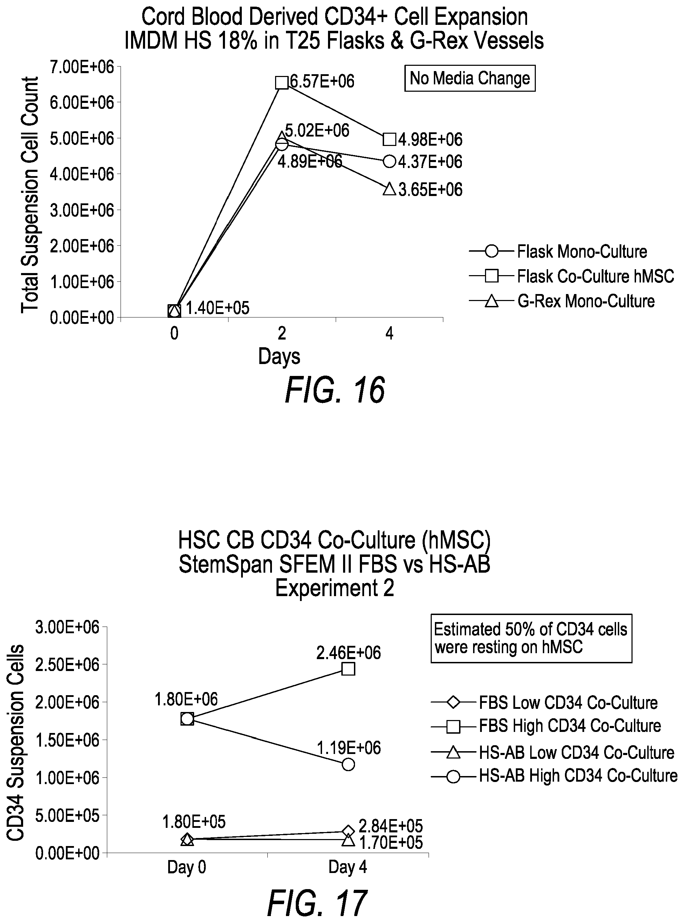

FIG. 15 illustrates a graph showing cell counts from Experiment 1 with FBS. FIG. 16 illustrates a graph showing cell counts from Experiment 1 with Human Serum.

Both the FBS and HS supplemented IMDM demonstrate an increase in cell count by Day-2. However, there is also a decrease in cell concentration by Day-4. There is also reduction in cell membrane integrity from Day-2 to Day-4 in both of the supplemented media from 87% to 76.4% with FBS and from 88.5% to 76.3% with HS. In our case, this may be due to the lack of a media change on Day-3 or the low cell seeding density of 2.00x10⁵/mL. In summary, the 20% FBS supplemented media may generate a 7.5-7.6 fold-increase in flask co-culture, 7.1 fold-increase in flask mono-culture, and a 7.0-7.2 fold-increase in the G-Rex-10 mono-culture by Day-4. By comparison, the 18% HS supplemented media may generate a 7.3-7.5 fold-increase in flask co-culture, 6.8 fold-increase in flask mono-culture, and a 6.7-6.9 fold-increase in the G-Rex10 mono-culture by Day-4.

Experiment 2 may evaluate both low and high seeding densities. FIG. 17 illustrates a graph showing CD34+ cells in both low and high seeding density conditions. Both the low (3.00x10⁴/mL) and high (3.00x10⁵/mL) CB CD34+ cell seeding density in 6 mL of StemSpan SFEM II supplemented with 20% FBS may generate an increase of suspension cell count on Day-4 of 57% and 36% respectively. In contrast, the 20% HS-AB supplemented media may generate a reduced suspension cell count over the same time 4-day culture period.

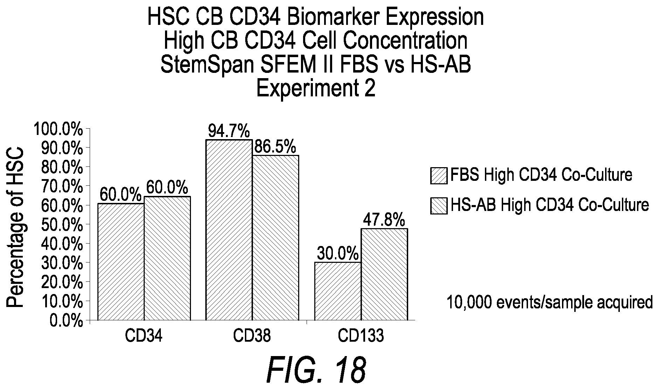

FIG. 18 illustrates a bar graph showing flow cytometry results. These flow cytometry results suggest that the majority of the CD34+ cells may also express the CD38+ biomarker in either the FBS or HS-AB supplemented media at the high cell seeding condition. The CD38 biomarker may be indicative of early progenitor CD34+ cell populations. More mature CD34+ cells may gradually become CD38 low/- during expansion in short-term culture after Day-4. CD133+CD34+ blood progenitor cells may be putitive markers of platelet engraftment in patients undergoing autologous peripheral blood stem cell transplantation. These data also indicate that the FBS and HS-AB supplemented media may support a CD133+ cell subpopulation, 30% and 47% respectively, in static co-culture.

### EXAMPLE 3

Below is an example of a protocol that may be used in embodiments. The protocol indicates possible modification from a conventional protocol(s) utilized when using a cell expansion system (CES).

### CES Cell Load Modification 3 - Load with Circ. Distribution & Rotation

A conventional Pre-selected MSC Expansion Protocol, may be performed with the following modifications shown in combined bold, underline, and italics below. Rotate the bioreactors 180 degrees and allow the cells to settle to the top of the hollow fiber membrane (HFM) for 5 minutes. Then rotate the bioreactor back to the "home" horizontal position and proceed with the expansion protocol. The rationale for the modification is to distribute the cells over the entire surface area of the bioreactor hollow fiber (FIGS. 10A-D).

### Day 0: Attach Feeder Cells with Rotation

Purpose: enables adherent cells to attach to the bioreactor membrane while allowing flow on the EC circulation loop. The pump flow rate to the IC loop is set to zero.

Prior to loading the cells into all a CES with Distribution and Rotation, install a Custom Task using the following existing/modified steps: Config>Task via the touch screen display or GUI. These solutions and corresponding volumes are based on the either default or modified settings for these custom tasks.

**Table 3: Solutions for Attaching Cells, Modification**

| | | |
|---|---|---|
| **Table 3: Solutions for Attach Cells** | | |

| **Bag** | **Solution in Bag** | **Volume** |
|---|---|---|
| Cell Inlet | None | N/A |
| Reagent | None | N/A |
| IC Media | ***Media with FBS or Serum-Free*** | 6 mL/hour |
| Wash | None | N/A |
| EC Media | None | N/A |

**Table 4: hMSC Feeder Layer Loading & Seeding**

| ***Table 4*: Custom 3 Task Settings to Load hMSCs** | | | | | |
|---|---|---|---|---|---|
| **Setting** | **Step 1** | **Step 2** | **Step 3** | **Step 4** | **Step 5** |
| IC Inlet | Cell | IC Media | None | None | IC Media |
| IC Inlet Rate | ***25*** | ***25*** | ***0.00*** | 0.00 | 0.1 |
| IC Circulation Rate | ***150*** | ***150*** | ***200*** | 0.00 | 20 |
| EC Inlet | None | None | None | IC Media | None |
| EC Inlet Rate | 0.00 | 0.00 | 0.00 | 0.1 | 0.00 |
| EC Circulation Rate | 30 | 30 | 30 | 30 | 30 |
| Outlet | EC Waste | EC Waste | EC Waste | EC Waste | IC Waste |
| Rocker Control | In Motion: (-90° to 180°) | In Motion: (-90° to 180°) | In Motion: (-90° to 180°) | ***Stationary: 180°*** | Stationary: 0° |
| Stop Condition | Empty Bag | IC Volume: 22 mL | ***Time: 2 min*** | ***Time: 1,440 min*** | Manual: |
| Estimated Fluid | Unknown | <0.1 L | Unknown | <0.2 L | Unknown |
| Omit or Include | Include | Include | Include | Include | Include |

Return to conventional cell feeding tasks on Day 2 or as needed and continue with the pre-cultured hMSC expansion protocol using Feed Cells Task.

### Day 5: Introduce CB CD34+ Suspension Cells with Rotation

IC/EC Exchange & Condition Media for CB CD34+ Cells.

Attach SCGM Base Media to IC Media line. Perform IC/EC Washout and Condition Media Tasks respectively.

Attach Cell Inlet bag with CB CD34+ cell suspension of SCGM (98 mL) + StemCell2MAX 100X supplement (2 mL) to Cell Inlet line of the System with a sterile welder.

**Table 5: CB CD34+ Cell Loading & Seeding**

| ***Table 5:* Custom Task 7 Settings to Load CB CD34+ Suspension Cells** | | | | |
|---|---|---|---|---|
| **Setting** | **Step 1** | **Step 2** | **Step 3** | **Step 4** |
| IC Inlet | Cell | IC Media | None | None |
| IC Inlet Rate | ***25*** | ***25*** | ***0.00*** | 0.00 |
| IC Circulation Rate | ***150*** | ***150*** | ***200*** | 0.00 |
| EC Inlet | None | None | None | IC Media |
| EC Inlet Rate | 0.00 | 0.00 | 0.00 | 0.1 |
| EC Circulation Rate | 30 | 30 | 30 | ***60*** |
| Outlet | EC Waste | EC Waste | EC Waste | ***EC Waste*** |
| Rocker Control | In Motion: (-90° to 180°) | In Motion: (-90° to 180°) | In Motion: (-90° to 180°) | Stationary: 0° |
| Stop Condition | Empty Bag | IC Volume: 22 mL | Time: 2 min | Manual: |
| Estimated Fluid | Unknown | <0.1 L | Unknown | Unknown |
| Omit or Include | Include | Include | Include | Include |

### Resuspension of Cells Prior to Harvest

The purpose of this Circulation Task is to resuspend those cells that may be attached to the hMSC feeder layer during co-culture prior to initiating the Harvest Task.

**Table 6: Circulation and Resuspension of Cells**

| ***Table 6*: Custom Task 1 Settings to Resuspend Settled CB CD34+ Cells** | |
|---|---|
| **Setting** | **Step 1** |
| IC Inlet | ***None*** |
| IC Inlet Rate | ***0*** |
| IC Circulation Rate | ***200*** |
| EC Inlet | ***None*** |
| EC Inlet Rate | ***0*** |
| EC Circulation Rate | ***60*** |
| Outlet | ***EC Waste*** |
| Rocker Control | ***In Motion:* *(-90°* to *180°)*** |
| | ***Dwell Time: 1 sec*** |
| Stop Condition | ***Time: 2 min*** |
| Estimated Fluid | Unknown |
| Omit or Include | Include |

### Harvest

CES Harvest Task with modification.

**Table 7: Harvest, Modification**

| ***Table 7*: Harvest, *Modified*** | |
|---|---|
| **Setting** | **Step 1** |
| IC Inlet | IC Media |
| IC Inlet Rate | 400 |
| IC Circulation Rate | -70 |
| EC Inlet | ***IC Media*** |
| EC Inlet Rate | 60 |
| EC Circulation Rate | 30 |
| Outlet | Harvest |
| Rocker Control | In Motion: (-90° to 180°) |
| | Dwell Time: 1 sec |
| Stop Condition | IC Volume: 378 mL |
| Estimated Fluid | IC Media: 0.5 L |
| Omit or Include | Include |

It will be apparent to those skilled in the art that various modifications and variations can be made to the methods and structure of the present invention without departing from its scope. Thus it should be understood that the present invention is not be limited to the specific examples given. Rather, the present invention is intended to cover modifications and variations within the scope of the following claims and their equivalents.

While example embodiments and applications of the present invention have been illustrated and described, it is to be understood that the invention is not limited to the precise configuration and resources described above. Various modifications, changes, and variations apparent to those skilled in the art may be made in the arrangement, operation, and details of the methods and systems of the present invention disclosed herein without departing from the scope of the present invention.

## Claims

1. A method of expanding cells, the method comprising:
coating with fibronectin an intracapillary side of a plurality of hollow fibers in a hollow fiber bioreactor;
introducing (808) a first plurality of cells comprising mesenchymal stem cells into the intracapillary side of the hollow fiber bioreactor;
exposing the first plurality of cells to first growth conditions, wherein the growth conditions comprise exposing the first plurality of cells to first growth media;
introducing a second plurality of cells comprising CD34+ cells into the intracapillary side of the hollow fiber bioreactor;
exposing the second plurality of cells to second growth conditions, wherein the second growth conditions comprise exposing the second plurality of cells to a plurality of growth factors including a combination of recombinant human Flt3 ligand (rhFlt-3L), recombinant human stem cell factor (rhSCF), recombinant human thrombopoietin (rhTPO) and recombinant human (rh) Glial-derived neurotrophic factors, wherein the exposing the second plurality of cells to second growth conditions comprises circulating base media through an extracapillary side of the hollow fiber bioreactor;
expanding the second plurality of cells in the plurality of hollow fibers in co-culture with the mesenchymal stem cells to generate a third plurality of expanded cells; and
removing the third plurality of expanded cells from the bioreactor.

2. The method of claim 1, wherein the exposing the first plurality of cells to first growth conditions comprises circulating base media through an extracapillary side of the hollow fiber bioreactor.

3. The method of claim 1, wherein the second plurality of cells is derived from cord blood.

4. The method of claim 1, wherein the second plurality of cells is added to the hollow fiber bioreactor without additional purification.

5. The method of claim 1, wherein the mesenchymal stem cells are bone marrow derived.

6. The method of claim 5, further comprising:
administering the third plurality of expanded cells to a patient to reconstitute hematopoiesis in the patient.

## Patentansprüche

1. Verfahren zum Expandieren von Zellen, wobei das Verfahren Folgendes umfasst:
Beschichten einer intrakapillären Seite einer Vielzahl von Hohlfasern in einem Hohlfaser-Bioreaktor mit Fibronectin;
Einbringen (808) einer ersten Vielzahl von Zellen, die mesenchymale Stammzellen umfassen, in die intrakapilläre Seite des Hohlfaser-Bioreaktors;
Aussetzen der ersten Vielzahl von Zellen gegenüber ersten Wachstumsbedingungen, wobei die Wachstumsbedingungen das Aussetzen der ersten Vielzahl von Zellen gegenüber ersten Wachstumsmedien umfassen;
Einbringen einer zweiten Vielzahl von Zellen, die CD34+-Zellen umfassen, in die intrakapilläre Seite des Hohlfaser-Bioreaktors;
Aussetzen der zweiten Vielzahl von Zellen gegenüber zweiten Wachstumsbedingungen, wobei die zweiten Wachstumsbedingungen das Aussetzen der zweiten Vielzahl von Zellen gegenüber einer Vielzahl von Wachstumsfaktoren umfassen, einschließlich einer Kombination aus rekombinantem humanem Flt3-Ligand (rhFlt-3L), rekombinantem humanem Stammzellfaktor (rhSCF), rekombinantem humanem Thrombopoietin (rhTPO) und rekombinanten humanen (rh) glialabgeleiteten neurotrophen Faktoren, wobei das Aussetzen der zweiten Vielzahl von Zellen gegenüber zweiten Wachstumsbedingungen das Zirkulieren von Basismedien durch eine extrakapilläre Seite des Hohlfaser-Bioreaktors umfasst;
Expandieren der zweiten Vielzahl von Zellen in der Vielzahl von Hohlfasern in Kokultur mit den mesenchymalen Stammzellen, um eine dritte Vielzahl von expandierten Zellen zu erzeugen; und
Entnehmen der dritten Vielzahl von expandierten Zellen aus dem Bioreaktor.

2. Verfahren nach Anspruch 1, wobei das Aussetzen der ersten Vielzahl von Zellen gegenüber ersten Wachstumsbedingungen das Zirkulieren von Basismedien durch eine extrakapilläre Seite des Hohlfaser-Bioreaktors umfasst.

3. Verfahren nach Anspruch 1, wobei die zweite Vielzahl von Zellen aus Nabelschnurblut stammt.

4. Verfahren nach Anspruch 1, wobei die zweite Vielzahl von Zellen dem Hohlfaser-Bioreaktor ohne zusätzliche Reinigung zugegeben wird.

5. Verfahren nach Anspruch 1, wobei die mesenchymalen Stammzellen aus dem Knochenmark stammen.

6. Verfahren nach Anspruch 5, ferner umfassend:
Verabreichen der dritten Vielzahl von expandierten Zellen an einen Patienten, um die Hämatopoese bei dem Patienten wiederherzustellen.

## Revendications

1. Procédé d'expansion de cellules, le procédé comprenant :
l'application d'un revêtement de fibronectine sur un côté intracapillaire d'une pluralité de fibres creuses dans un bioréacteur à fibres creuses ;
l'introduction (808) d'une première pluralité de cellules, comprenant des cellules souches mésenchymateuses, dans le côté intracapillaire du bioréacteur à fibres creuses ;
l'exposition de la première pluralité de cellules à des premières conditions de croissance, dans lequel les conditions de croissance comprennent l'exposition de la première pluralité de cellules à un premier milieu de croissance ;
l'introduction d'une deuxième pluralité de cellules, comprenant des cellules CD34+, dans le côté intracapillaire du bioréacteur à fibres creuses ;
l'exposition de la deuxième pluralité de cellules à des secondes conditions de croissance, dans lequel les secondes conditions de croissance comprennent l'exposition de la deuxième pluralité de cellules à une pluralité de facteurs de croissance incluant une combination de ligand Flt3 humain recombinant (rhFlt-3L), de facteur humain recombinant de cellules souches (rhSCF), de thrombopoïétine humaine recombinante (rhTPO), et de facteurs neurotrophiques humains recombinants dérivés de cellules gliales (rh), dans lequel l'exposition de la deuxième pluralité de cellules à des secondes conditions de croissance comprend la mise en circulation d'un milieu de base à travers un côté extracapillaire du bioréacteur à fibres creuses ;
l'expansion de la deuxième pluralité de cellules dans la pluralité de fibres creuses en coculture avec les cellules souches mésenchymateuses pour générer une troisième pluralité de cellules expansées ; et
le retrait de la troisième pluralité de cellules expansées du bioréacteur.

2. Procédé de la revendication 1, dans lequel l'exposition de la première pluralité de cellules à des premières conditions de croissance comprend la mise en circulation d'un milieu de base à travers un côté extracapillaire du bioréacteur à fibres creuses.

3. Procédé de la revendication 1, dans lequel la deuxième pluralité de cellules est dérivée de sang de cordon.

4. Procédé de la revendication 1, dans lequel la deuxième pluralité de cellules est ajoutée au bioréacteur à fibres creuses sans purification supplémentaire.

5. Procédé de la revendication 1, dans lequel les cellules souches mésenchymateuses sont dérivées de moelle osseuse.

6. Procédé de la revendication 5, comprenant en outre :
l'administration de la troisième pluralité de cellules expansées à un patient pour reconstituer l'hématopoïèse chez le patient.
